# EUROPÄISCHE PATENTSCHRIFT

(11) **EP 3 463 583 B1**
(45) Veröffentlichungstag und Bekanntmachung des Hinweises auf die Patenterteilung: **03.03.2021**
(21) Anmeldenummer: 17723938.1
(22) Anmeldetag: 27.04.2017
(51) Int. Cl.: A61Q 5/08, A61Q 5/10, A61K 8/22, A61K 8/92, A61K 8/49, A61K 8/43, A61K 8/44, A61K 8/365, A61K 8/362, A61K 8/36

(54) **PASTENFÖRMIGES BLONDIERMITTEL**
PASTY HAIR LIGHTENING AGENT
PÂTE D'ECLAIRCIR DE CHEVEUX

(30) Priorität: 31.05.2016 DE 102016209464
(43) Veröffentlichungstag der Anmeldung: 10.04.2019
(73) Patentinhaber: Henkel AG & Co. KGaA, 40589 Düsseldorf (DE)
(72) Erfinder: ANDERHEGGEN, Bernd, 41189 Mönchengladbach (DE); MÜLLER, Burkhard, 40221 Düsseldorf (DE)
(86) Internationale Anmeldenummer: PCT/EP2017/060112
(87) Internationale Veröffentlichungsnummer: WO 2017/207191

(56) Entgegenhaltungen:
- WO-A1-2005/072689
- DE-A1- 10 051 774
- DE-A1-102005 053 850

## Beschreibung

Die vorliegende Erfindung betrifft Blondierpasten, die als Mittel zum Aufhellen von keratinischen Fasern, insbesondere menschlichen Haaren, dienen. Weiterhin betrifft die vorliegende Erfindung die Verwendung einer erfindungsgemäßen Blondierpaste zur schonenden Blondierung bzw. oxidativen Aufhellung von menschlichen Haaren sowie eine Mehrkomponentenverpackungseinheit (Kit-of-Parts) zur Aufhellung keratinischer Fasern, die eine erfindungsgemäße Blondierpaste und davon getrennt eine Oxidationsmittelzubereitung umfasst.

Weiterhin wird ein Verfahren zur oxidativen Aufhellung von keratinischen Fasern unter Verwendung der genannten Blondierpaste und des diese Blondierpaste umfassenden Kits beschrieben. Bei einer Blondierpaste handelt es sich eine wasserfreie, pastenförmige Oxidationsmittelzubereitung, enthaltend mindestens ein Persalz oder Percarbonat.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein. Für das Erzielen eines stärkeren Blondiereffekts wird üblicherweise eine Mischung aus Wasserstoffperoxid und mindestens einer Verbindung, ausgewählt aus Percarbonaten und Persalzen, insbesondere Peroxodisulfatsalzen und/oder Peroxomonosulfatsalzen, eingesetzt. Zur Verstärkung der Blondierwirkung enthalten die Mittel höhere Einsatzkonzentrationen an Wasserstoffperoxid und Percarbonaten oder Persalzen, insbesondere Persulfaten. Dunkles, dunkelbraunes oder schwarzes Haar lässt sich so in einem Schritt um 4 bis 6 Nuancen aufhellen. Das Wasserstoffperoxid und die Percarbonate oder Persalze werden bis zur Anwendung getrennt voneinander aufbewahrt, um die Percarbonate oder Persalze nicht vorzeitig zu deaktivieren. Die Wasserstoffperoxid-Komponente, die eine wässrige Lösung von Wasserstoffperoxid umfasst, weist zur Stabilisierung des Wasserstoffperoxids einen sauren pH-Wert, insbesondere einen pH-Wert von 2,5 bis 5,5, insbesondere von 3 bis 5, auf, jeweils gemessen bei 20°C.

Für die Melanin abbauende Wirkung des Wasserstoffperoxids und die Blondierwirkung auf der keratinischen Faser ist es jedoch vorteilhaft, wenn die Anwendungsmischung aus Wasserstoffperoxid-Lösung und Persalz einen alkalischen pH-Wert besitzt, der vorzugsweise im Bereich von 8 bis 12, besonders bevorzugt im Bereich von 8,5 bis 11,5, außerordentlich bevorzugt im Bereich von 9 bis 10,5 liegt, jeweils gemessen bei 20°C.

Zur Einstellung eines alkalischen pH-Werts der aufhellenden Anwendungsmischung gibt es mehrere Möglichkeiten:
Die Blondierpaste enthält neben dem mindestens einen Persalz oder Percarbonat mindestens ein Alkalisierungsmittel in einer solchen Gesamtmenge, dass die Anwendungsmischung den gewünschten alkalischen pH-Wert aufweist; oder
die Wasserstoffperoxidlösung wird nicht nur mit der Blondierpaste, sondern zusätzlich mit einer Alkalisierungsmittelzubereitung zur Anwendungsmischung kombiniert.

Blondierpasten sind Suspensionen von pulverförmigen Persalzen oder Percarbonaten und optional Alkalisierungsmittel(n), die pulverförmig sind, in einem Öl oder einer Ölmischung, die im Wesentlichen wasserfrei und optional verdickt ist.

Setzt man der Alkalisierungsmittelzubereitung und/oder der Blondierpaste Oxidationsfarbstoffvorprodukt(e) und/oder direktziehende(n) Farbstoff(e) zu, so kann das Haar gleichzeitig gefärbt werden. Entsprechende 3-Komponenten-Haarfärbemittel werden insbesondere für Verbraucher mit sehr dunklem Melanin-reichen Haar angeboten.

Mit der Aufhellung geht jedoch auch eine Schädigung des Haares einher, da nicht nur die Farbstoffe des Haares, sondern auch die Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Persalze oder Percarbonate ist, desto stärkere Schädigungen werden daher in der Regel auf den Keratinfasern hervorgerufen.

Um die Haarschädigung zu minimieren und den schädigenden Effekt der Oxidationsmittel wenigstens teilweise auszugleichen, kann man Persalz-haltige Haaraufhellungs- und -färbemittel mit einem höheren Gehalt an Ölen formulieren. Im Stand der Technik sind Blondiermittelsuspensionen, die wasserfreie Suspensionen von feinteiligen, bei 25°C und 1013 mbar festen Persalzen oder Percarbonaten in einem Öl oder einer Ölmischung darstellen, die gegebenenfalls mit einem Ölgeliermittel verdickt sein können, beispielsweise beschrieben in EP 0778020, EP 1034777 EP 1380287 und WO 2009/134875 A1.

DE102005053850A1 offenbart wasserfreie Blondierpasten mit einer festen Peroxoverbindung, einem Alkalisierungsmittel und zusätzlich einem nicht hydroxylierten Fettsäureester mit einem Schmelzpunkt von höchstens 50°C, und mindestens einer C₁₀- bis C₃₀-Fettsäure mit mindestens einer zusätzlichen Hydroxygruppe, die sich gut mit einer Wasserstoffperoxidlösung vermischen lassen. DE10051774A1 offenbart Haarpflegemittel, die durch einen Gehalt an Bernsteinsäure die durch eine reduktive Haarbehandlung erzeugte Haarschädigung reparieren können. WO2005072689A1 offenbart, dass der Ölgehalt in halbfesten Bleichpasten dazu beiträgt, den Zugfestigkeitsverlust des Haars durch die oxidative Behandlung zu verringern.

Der vorliegenden Erfindung lag die Aufgabe zu Grunde, Mittel zum Aufhellen bzw. Blondieren von keratinischen Fasern, insbesondere menschlichen Haaren, bereitzustellen, die die Keratinfasern möglichst wenig schädigen und die einfach herzustellen und zu handhaben sind.

Überraschend wurden diese Aufgaben durch den Gegenstand der Ansprüche gelöst.

Unter keratinhaltigen bzw. keratinischen Fasern werden erfindungsgemäß Pelze, Wolle, Federn und insbesondere menschliche Haare verstanden. Obwohl die erfindungsgemäßen Mittel in erster Linie zum Blondieren und/oder Aufhellen von keratinhaltigen Fasern geeignet sind, steht prinzipiell einer Verwendung auch auf anderen Gebieten nichts entgegen.

Ein geeigneter Parameter für die Quantifizierung der Faserschädigung, insbesondere der Haarschädigung, ist die Messung der Zugfestigkeit (Young-Modul) der Keratinfasern.

WO 2005/115314A1 offenbart ein Verfahren zur Restrukturierung keratinischer Fasern, bei dem die Keratinfasern mit Cystin und mit mindestens einer Dicarbonsäure mit 2 bis 10 Kohlenstoffatomen in Kontakt gebracht werden, wobei bevorzugte Dicarbonsäuren ausgewählt sind aus Oxalsäure, Malonsäure, Bernsteinsäure, Glutarsäure, Adipinsäure, Pimelinsäure, Azelainsäure, Maleinsäure, Fumarsäure und Sorbinsäure und Bernsteinsäure besonders bevorzugt ist. DE 10051774 A1 beschreibt die Verwendung kurzkettiger Carbonsäuren mit einem Molekulargewicht unter 750 g/mol in kosmetischen Mitteln als Wirkstoff zur Restrukturierung keratinischer Fasern. EP1174112A offenbart Haarbehandlungsmittel, die neben einer organischen Säure als weitere zwingende Bestandteile ein organisches Lösungsmittel, ein kationisches Tensid und einen höheren Alkohol enthalten und zur Reparatur von Poren in Haaren dienen.

Ein erster Gegenstand der vorliegenden Erfindung ist eine Blondierpaste, enthaltend
a) mindestens ein Oxidationsmittel, ausgewählt aus Natriumpercarbonaten und anorganischen Salzen einer Peroxoschwefelsäure, und sowie Mischungen hiervon,
b) weiterhin eine Dicarbonsäure mit 2 bis 10 Kohlenstoffatomen, ausgewählt aus Bernsteinsäure und/oder mindestens einem Salz der Bernsteinsäure,
c) weiterhin Arginin und Lysin oder mindestens eines der Salze dieser Aminosäuren,
d) mindestens ein Öl in einer Gesamtmenge von 16-60 Gew.-%, bevorzugt 20 - 50 Gew.-%, besonders bevorzugt 25 - 45 Gew.-%, jeweils bezogen auf das Gewicht der Blondierpaste, und
e) 0 bis 4 Gew.-% Wasser, bezogen auf das Gewicht der Blondierpaste.

Unter den Begriffen "Paste" oder "pastenförmig" ist erfindungsgemäß eine Darreichungsform zu verstehen, die bei 20°C und 1013 mbar eine Viskosität im Bereich von 200.000 bis 1.600.000 mPas, bevorzugt 250.000 bis 1.400.000 mPas, besonders bevorzugt 300.000 bis 1.000.000 mPas, außerordentlich bevorzugt 400.000 bis 750.000 mPas, aufweist.

Die Bestimmung der Pastenviskosität erfolgt bevorzugt mittels Brookfield; Gerät RVDV II+; Spindel Nr. 96, 4 Umdrehungen pro Minute, bei 20°C.

Sofern nicht anders angegeben, beziehen sich alle Temperaturangaben auf einen Druck von 1013 mbar.

Die erfindungsgemäße Blondierpaste enthält als ersten wesentlichen Bestandteil mindestens ein Oxidationsmittel, das aus Natriumpercarbonaten und anorganischen Salzen einer Peroxoschwefelsäure sowie Mischungen hiervon ausgewählt ist.

Unter Natriumpercarbonaten werden Natriumcarbonat-Wasserstoffperoxid-Komplexe verstanden. Handelsübliches Natriumpercarbonat hat die durchschnittliche Zusammensetzung 2 Na₂CO₃ . 3 H₂O₂. Natriumpercarbonat liegt als weißes, wasserlösliches Pulver vor, das leicht in Natriumcarbonat und bleichend und oxidierend wirkenden "aktiven" Sauerstoff zerfällt.

Unter Peroxoschwefelsäuren werden Peroxodischwefelsäure und Peroxomonoschwefelsäure (Caro'sche Säure) verstanden.

Vorzugsweise ist das mindestens eine anorganische Salz einer Peroxoschwefelsäure ausgewählt aus Ammoniumperoxodisulfat, Alkalimetallperoxodisulfaten, Ammoniumperoxomonosulfat, Alkalimetallperoxomonosulfaten und Alkalimetallhydrogenperoxomonosulfaten. Besonders bevorzugt sind Ammoniumperoxodisulfat, Kaliumperoxodisulfat, Natriumperoxodisulfat und Kaliumhydrogenperoxomonosulfat. Weiterhin hat es sich bei den Arbeiten zur vorliegenden Erfindung als besonders bevorzugt erwiesen, wenn die erfindungsgemäße Blondierpaste mindestens zwei verschiedene Peroxodisulfate enthält. Bevorzugte Peroxodisulfatsalze sind dabei Kombinationen aus Ammoniumperoxodisulfat und Kaliumperoxodisulfat und/oder Natriumperoxodisulfat.

Bevorzugte erfindungsgemäße Blondierpasten enthalten mindestens ein Oxidationsmittel, das aus Natriumpercarbonaten und anorganischen Salzen einer Peroxoschwefelsäure sowie Mischungen hiervon ausgewählt ist, in einer Gesamtmenge von 2,5 - 65 Gew.-%, bevorzugt 10-60 Gew.-%, weiter bevorzugt 20 - 55 Gew.-%, besonders bevorzugt 25 - 50 Gew.-% und insbesondere 30 - 45 Gew.-%, jeweils bezogen auf das Gewicht der Blondierpaste.

Die erfindungsgemäße Blondierpaste enthält als zweiten wesentlichen Bestandteil weiterhin eine Dicarbonsäure mit 2 bis 10 Kohlenstoffatomen, ausgewählt aus Bernsteinsäure und/oder mindestens einem Salz der Bernsteinsäure.

Erfindungsgemäß geeignete Salze der Dicarbonsäure mit 2 bis 10 Kohlenstoffatomen sind ausgewählt aus den Mono- und Disalzen der Anionen von Bernsteinsäure mit Ammoniumionen, Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Bernsteinsäure weist bei 1013 mbar einen Schmelzpunkt im Bereich von 185 - 187°C auf, ist also bei 20°C ein Feststoff. Erfindungsgemäß geeignete Salze der Bernsteinsäure sind ausgewählt aus den Succinaten und Hydrogensuccinaten von Ammoniumionen, Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze. Die genannten Salze der Bernsteinsäure können auch gebundenes Kristallwasser enthalten, insbesondere das Natriumsuccinathexahydrat, das erfindungsgemäß besonders bevorzugt ist.

Erfindungsgemäß bevorzugte Blondierpasten enthalten die Dicarbonsäure mit 2 bis 10 Kohlenstoffatomen, ausgewählt aus Bernsteinsäure und/oder mindestens einem Salz der Bernsteinsäure, in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Blondierpaste, wobei der Gesamtgehalt an Dicarbonsäuren mit 2 bis 10 Kohlenstoffatomen, ausgewählt aus Bernsteinsäure, Äpfelsäure, Oxalsäure, Malonsäure, Adipinsäure, Pimelinsäure, Korksäure, Azelainsäure, Sebacinsäure, Maleinsäure, Fumarsäure, D-Weinsäure, L-Weinsäure, meso-Weinsäure, Traubensäure, alpha-Ketoglutarsäure, beta-Ketoglutarsäure, Oxalessigsäure und den Salzen dieser Säuren, in einer auf die Masse an freier Dicarbonsäure umgerechneten Gesamtmenge 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Blondierpaste, beträgt.

Weitere erfindungsgemäß bevorzugte Blondierpasten enthalten Bernsteinsäure und/oder mindestens ein Salz der Bernsteinsäure in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Blondierpaste.

Die erfindungsgemäße Blondierpaste enthält als dritten wesentlichen Bestandteil weiterhin Arginin und Lysin oder mindestens eines der Salze dieser Aminosäuren. Unter den erfindungsgemäß bevorzugt geeigneten Salzen von Arginin und Lysin sind die Ammoniumsalze, Alkalimetallsalze und Erdalkalimetallsalze, insbesondere die Lithium-, Natrium-, Kalium-, Magnesium- und Calciumsalze, außerdem die Hydrohalogenide, insbesondere die Hydrochloride, sowie Mischungen dieser Salze. Ein erfindungsgemäß besonders bevorzugtes Aminosäuresalz ist Lysinhydrochlorid. Die erfindungsgemäß geeigneten Aminosäuren, ausgewählt aus Arginin und Lysin und deren Salzen, können auch Kristallwasser enthalten.

Erfindungsgemäß bevorzugte Blondierpasten enthalten Arginin und Lysin oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,3 - 2,5 Gew.-%, außerordentlich bevorzugt 1 - 2 Gew.-%, jeweils bezogen auf das Gewicht der Blondierpaste, wobei der Gesamtgehalt an den Aminosäuren Arginin und Lysin, ggf. Histidin, und den Salzen dieser Aminosäuren in einer auf die Masse an freier Aminosäure umgerechneten Gesamtmenge 0,1 - 7 Gew.- %, bevorzugt 0,2 - 5 Gew.-%, besonders bevorzugt 0,5 - 2,5 Gew.-%, außerordentlich bevorzugt 1 - 2 Gew.-%, jeweils bezogen auf das Gewicht der Blondierpaste, beträgt.

Erfindungsgemäß besonders bevorzugte Blondierpasten enthalten Bernsteinsäure und/oder ein mindestens ein Bernsteinsäuresalz in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Blondierpaste, sowie weiterhin Arginin und Lysin, ggf. Histidin, oder mindestens ein Salz dieser Aminosäuren, in einer auf die Masse an freier Aminosäure umgerechneten Gesamtmenge von 0,1 - 7 Gew.-%, bevorzugt 0,2 - 5 Gew.-%, besonders bevorzugt 0,3 - 2,5 Gew.-%, außerordentlich bevorzugt 1 - 2 Gew.-%, jeweils bezogen auf das Gewicht der Blondierpaste.

Die erfindungsgemäßen Blondierpasten weisen einen Wassergehalt von 0 bis 4 Gew.-%, bevorzugt 0,1 bis 2 Gew.-%, besonders bevorzugt 0,2 bis 0,7 Gew.-%, Wasser auf, jeweils bezogen auf das Gewicht der Blondierpaste. Diese Angaben beziehen sich auf den Gehalt an freiem Wasser. Nicht berücksichtigt ist der Gehalt an molekular gebundenem Wasser oder Kristallwasser, den einzelne Pastenbestandteile aufweisen können.

Der Wassergehalt kann mittels Karl-Fischer-Titration bestimmt werden, beispielsweise in Anlehnung an ISO 4317 (Version 2011-12).

Erfindungsgemäße und erfindungsgemäß bevorzugte Blondierpasten enthalten mindestens ein Öl in einer Gesamtmenge von 16-60 Gew.-%, bevorzugt 20 - 50 Gew.-%, besonders bevorzugt 25 - 45 Gew.-%, jeweils bezogen auf das Gewicht der Blondierpaste.

Das mindestens eine Öl, das als Trägermedium in den erfindungsgemäßen Blondierpasten enthalten ist, ist insbesondere ausgewählt aus Paraffinöl, Siliconöl oder Esteröl sowie Mischungen dieser Öle.

Weitere erfindungsgemäß bevorzugte Öle sind ausgewählt aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, weiterhin ausgewählt aus C₈-C,₆-Isoparaffinen, insbesondere aus Isodecan, Isododecan, Isotetradecan und Isohexadecan sowie Mischungen hiervon, sowie 1,3-Di-(2-ethylhexyl)-cyclohexan.

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Bevorzugte Alkoholöle sind 2-Hexyldecanol, 2-Octyldodecanol, 2-Ethylhexylalkohol und Isostearylalkohol, sowie Mischungen hiervon.

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/Caprylic Triglycerides.

Weitere erfindungsgemäß besonders bevorzugte kosmetische Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen bevorzugt 2-Hexyldecylstearat, 2-Hexyldecyllaurat, Isodecylneopentanoat, Isononylisononanoat, 2-Ethylhexylpalmitat und 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat und Ethylenglycoldioleat.

Weitere erfindungsgemäß bevorzugte kosmetische Öle sind ausgewählt aus den Anlagerungsprodukten von 1 bis 5 Propylenoxid-Einheiten an ein- oder mehrwertige C₈-₂₂-Alkanole, wie Octanol, Decanol, Decandiol, Laurylalkohol, Myristylalkohol und Stearylalkohol, z. B. PPG-2-Myristylether und PPG-3-Myristylether. Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C₃-₂₂-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.

Weitere erfindungsgemäß bevorzugte kosmetische Öle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure, z. B. C₁₂-C₁₅-Alkyllactat.

Weitere erfindungsgemäß bevorzugte kosmetische Öle sind ausgewählt aus den symmetrischen, unsymmetrischen oder cyclischen Estern der Kohlensäure mit C₃-₂₂-Alkanolen, C₃-₂₂-Alkandiolen oder C₃-₂₂-Alkantriolen, z. B. Dicaprylylcarbonat, oder die Ester gemäß DE 19756454 A1, insbesondere Glycerincarbonat.

Weitere kosmetische Öle, die erfindungsgemäß geeignet sind, sind ausgewählt aus den Siliconölen, zu denen z. B. Dialkyl- und Alkylarylsiloxane, wie beispielsweise Decamethylcyclopentasiloxan, Dodecamethylcyclohexasiloxan, Dimethylpolysiloxan und Methylphenylpolysiloxan, aber auch Hexamethyldisiloxan, Octamethyltrisiloxan und Decamethyltetrasiloxan zählen.

Es kann erfindungsgemäß außerordentlich bevorzugt sein, Mischungen der vorgenannten Öle einzusetzen.

Bevorzugte erfindungsgemäße Blondierpasten sind dadurch gekennzeichnet, dass das kosmetische Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈-₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈-₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈-₂₂-Alkanole; den Anlagerungsprodukten von mindestens 6 Ethylenoxid- und/oder Propylenoxid-Einheiten an ein- oder mehrwertige C₃-₂₂-Alkanole; den C₈-C₂₂-Fettalkoholestern einwertiger oder mehrwertiger C2-C7-Hydroxycarbonsäuren; den symmetrischen, unsymmetrischen oder cyclischen Estern der Kohlensäure mit C₃-₂₂-Alkanolen, C₃-₂₂-Alkandiolen oder C₃-₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Atkanolen oder mit mehrwertigen linearen oder verzweigten C₂-C₆-Alkanolen; Siliconölen sowie Mischungen der vorgenannten Substanzen, bevorzugt in einer Gesamtmenge von 16-60 Gew.-%, besonders bevorzugt 20 - 50 Gew.-%, außerordentlich bevorzugt 25 - 45 Gew.-%, jeweils bezogen auf das Gewicht der Blondierpaste.

Erfindungsgemäß bevorzugte Blondierpasten enthalten zusätzlich mindestens ein anorganisches, bei 20°C und 1013 mbar festes Alkalisierungsmittel, das bevorzugt in einer Gesamtmenge von 0,5 - 15 Gew.-%, bevorzugt 1 - 10 Gew.-%, besonders bevorzugt 2-8 Gew.%, außerordentlich bevorzugt 3 - 7 Gew.-%, jeweils bezogen auf das Gewicht der Blondierpaste, enthalten ist. Erfindungsgemäß besonders bevorzugte anorganische, bei 20°C und 1013 mbar feste Alkalisierungsmittel sind ausgewählt aus Alkalimetallsilikaten, Erdalkalimetallsilikaten, Erdalkalimetallhydroxidcarbonaten, Erdalkalimetallcarbonaten, Alkalimetallmetasilikaten, Erdalkalimetallmetasilikaten, Alkalimetallhydroxiden, Erdalkalimetallhydroxiden, (Erd-)Alkalimetallphosphaten und (Erd-)Alkalimetallhydrogenphosphaten sowie Mischungen dieser Substanzen. Erfindungsgemäß besonders bevorzugte anorganische, bei 20°C und 1013 mbar feste Alkalisierungsmittel sind ausgewählt aus Natriummetasilikaten mit einem molaren SiO₂/Na₂O-Verhältnis von 0,8 - 1,2, bevorzugt von 0,9 - 1,1, außerordentlich bevorzugt von 1.

Erfindungsgemäß besonders bevorzugte Blondierpasten enthalten, jeweils bezogen auf ihr Gesamtgewicht, 0,5 - 15 Gew.-%, bevorzugt 1-10 Gew.-%, besonders bevorzugt 2-8 Gew.%, außerordentlich bevorzugt 3-7 Gew.-%, jeweils bezogen auf das Gewicht der Blondierpaste, Natriummetasilikate mit einem molaren SiO₂/Na₂O-Verhältnis von 0,8 - 1,2, bevorzugt von 0,9 - 1,1, außerordentlich bevorzugt von 1, als anorganische, bei 20°C und 1013 mbar feste Alkalisierungsmittel.

Um eine möglichst gleichmäßige, lagerstabile Suspension der obligatorischen Bestandteile a), b) und c) und ggf. weiterer Bestandteile, die im Trägeröl unlöslich sind, zu gewährleisten, enthalten erfindungsgemäß bevorzugte Blondierpasten mindestens eine Substanz, die die Ölphase verdickt. Bevorzugte Verdickungsmittel für die Ölphase sind ausgewählt aus Copolymeren von C2-C4-Alken und Styrol, linearen gesättigten 1-Alkanolen mit 12 - 30 Kohlenstoffatomen, Estern aus gesättigten verzweigten oder unverzweigten Alkancarbonsäuren mit 12 bis 24 C-Atomen und gesättigten verzweigten oder unverzweigten Alkoholen mit 16 bis 50 C-Atomen, wobei die Ester einen Schmelzpunkt im Bereich von 50°C bis 110°C aufweisen, Triglyceriden gesättigter und gegebenenfalls hydroxylierter C₁₂₋₃₀-Fettsäuren, wobei die Triglyceride einen Schmelzpunkt im Bereich von 50°C bis 110°C aufweisen, sowie Mischungen der vorgenannten Substanzen.

Erfindungsgemäß bevorzugte Blondierpasten enthalten mindestens eine Substanz, die die Ölphase verdickt, in einer Gesamtmenge von 1-15 Gew.-%, bevorzugt 2-10 Gew.-%, besonders bevorzugt 3-8 Gew.-%, besonders bevorzugt 4 - 6,5 Gew.-%, jeweils bezogen auf das Gewicht der Blondierpaste.

Weitere erfindungsgemäß bevorzugte Blondierpasten enthalten mindestens eine Substanz, die die Ölphase verdickt, in einer Gesamtmenge von 1-15 Gew.-%, bevorzugt 2-10 Gew.-%, besonders bevorzugt 3-8 Gew.-%, besonders bevorzugt 4 - 6,5 Gew.-%, jeweils bezogen auf das Gewicht der Blondierpaste, wobei die mindestens eine Substanz, die die Ölphase verdickt, ausgewählt ist aus Copolymeren von C2-C4-Alken und Styrol, linearen gesättigten 1-Alkanolen mit 12-30 Kohlenstoffatomen, Estern aus gesättigten verzweigten oder unverzweigten Alkancarbonsäuren mit 12 bis 24 C-Atomen und gesättigten verzweigten oder unverzweigten Alkoholen mit 16 bis 50 C-Atomen, wobei die Ester einen Schmelzpunkt im Bereich von 50°C bis 110°C aufweisen, Triglyceriden gesättigter und gegebenenfalls hydroxylierter C₁₂-₃₀-Fettsäuren, wobei die Triglyceride einen Schmelzpunkt im Bereich von 50°C bis 110°C aufweisen, sowie Mischungen der vorgenannten Substanzen.

Erfindungsgemäß bevorzugte Copolymere von C2-C4-Alken und Styrol, die die Ölphase verdicken, sind in erfindungsgemäß bevorzugten Blondierpasten in einer Gesamtmenge von 0,1 - 1,5 Gew.- %, bevorzugt 0,2 - 1 Gew.-%, besonders bevorzugt 0,3 - 0,8 Gew.-%, außerordentlich bevorzugt 0,4 - 0,6 Gew.-%, enthalten, jeweils bezogen auf das Gewicht der Blondierpaste.

Erfindungsgemäß bevorzugte Copolymere von C2-C4-Alken und Styrol, die die Ölphase verdicken, sind ausgewählt aus Copolymeren von Ethylen/Propylen/Styrol, Copolymeren von Butylen/Ethylen/ Styrol, Copolymeren von Butylen/Propylen/Styrol sowie Mischungen dieser Copolymere.

Vorzugsweise sind die genannten Copolymere von C2-C4-Alken und Styrol keine Copolymere, in denen die Monomereinheiten statistisch verteilt sind, sondern Blockcopolymere, besonders bevorzugt Diblockcopolymere und Triblockcopolymere. Solche Blockcopolymere weisen "harte" Segmente aus Polystyrol und "weiche" Segmente aus Ethylen/Propylen bzw. Ethylen/Butylen bzw. Propylen/ Butylen auf. Die einzelnen Blöcke können dabei 10 bis 10000, vorzugsweise 50 bis 5000 und insbesondere 100 bis 500 Monomere umfassen. Bevorzugte Diblockcopolymere sind Styrol-Ethylenpropylen (S-EP) und Styrol-Ethylenbutylen (S-EB); bevorzugte Triblockcopolymere sind Styrol-Ethylenpropylen-Styrol (S-EP-S) und Styrol-Ethylenbutylen-Styrol (S-EB-S). Erfindungsgemäß besonders bevorzugt ist es, Mischungen von Diblock- und Triblock-Copolymeren einzusetzen, wobei sich Mischungen von Styrol-Ethylenpropylen (S-EP) und Styrol-Ethylenpropylen-Styrol (S-EP-S) als besonders bevorzugt herausgestellt haben. Ganz besonders bevorzugt beträgt hierbei der Anteil an Diblockcopolymeren 10 bis 90 Gew.-% und der Anteil an Triblockcopolymeren 90 bis 10 Gew.-%, jeweils bezogen auf das Gewicht der Polymermischung.

Erfindungsgemäß bevorzugte Blondierpasten enthalten mindestens ein Copolymer von C2-C4-Alken und Styrol, das die Ölphase verdickt und das ausgewählt ist aus Copolymeren von Ethylen/ Propylen/Styrol, Copolymeren von Butylen/Ethylen/ Styrol, Copolymeren von Butylen/Propylen/ Styrol sowie Mischungen dieser Copolymere, in einer Gesamtmenge von 0,1 - 1,5 Gew.-%, bevorzugt 0,2 - 1 Gew.-%, besonders bevorzugt 0,3 - 0,8 Gew.-%, außerordentlich bevorzugt 0,4 - 0,6 Gew.-%, enthalten, jeweils bezogen auf das Gewicht der Blondierpaste.

Erfindungsgemäß besonders bevorzugte Blondierpasten enthalten eine Kombination aus Ethylen/ Propylen/Styrol-Copolymer und Butylen/Ethylen/Styrol-Copolymer, besonders bevorzugt in einer Gesamtmenge von 0,1 - 1,5 Gew.-%, bevorzugt 0,2 - 1 Gew.-%, besonders bevorzugt 0,3 - 0,8 Gew.-%, außerordentlich bevorzugt 0,4 - 0,6 Gew.-%, enthalten, jeweils bezogen auf das Gewicht der Blondierpaste.

Erfindungsgemäß bevorzugte lineare gesättigte 1-Alkanole mit 12-30 Kohlenstoffatomen, die die Ölphase verdicken, sind in erfindungsgemäß bevorzugten Blondierpasten in einer Gesamtmenge von 0,1 - 10 Gew.-%, bevorzugt 0,5 - 8 Gew.-%, besonders bevorzugt 1 - 7 Gew.-%, außerordentlich bevorzugt 2-5 Gew.-%, enthalten, jeweils bezogen auf das Gewicht der Blondierpaste. Erfindungsgemäß bevorzugte lineare gesättigte 1-Alkanole mit 12-30 Kohlenstoffatomen sind ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/ Stearylalkohol-Mischungen.

Erfindungsgemäß besonders bevorzugte Blondierpasten enthalten mindestens ein lineares gesättigtes 1-Alkanol mit 12-30 Kohlenstoffatomen, ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/Stearylalkohol-Mischungen, in einer Gesamtmenge von 0,1 - 10 Gew.-%, bevorzugt 0,5 - 8 Gew.-%, besonders bevorzugt 1 - 7 Gew.-%, außerordentlich bevorzugt 2-5 Gew.-%, enthalten, jeweils bezogen auf das Gewicht der Blondierpaste.

Erfindungsgemäß bevorzugte Ester aus gesättigten verzweigten oder unverzweigten Alkancarbonsäuren mit 12 bis 24 C-Atomen und gesättigten verzweigten oder unverzweigten Alkoholen mit 16 bis 50 C-Atomen mit einem Schmelzpunkt im Bereich von 50°C bis 110°C, die die Ölphase verdicken, sind in erfindungsgemäß bevorzugten Blondierpasten in einer Gesamtmenge von 0,1 - 5 Gew.-%, bevorzugt 0,2 - 4 Gew.-%, besonders bevorzugt 0,3 - 2 Gew.-%, außerordentlich bevorzugt 0,4 - 1 Gew.-%, enthalten, jeweils bezogen auf das Gewicht der Blondierpaste.

Erfindungsgemäß bevorzugte Ester aus gesättigten verzweigten oder unverzweigten Alkancarbonsäuren mit 12 bis 24 C-Atomen und gesättigten verzweigten oder unverzweigten Alkoholen mit 16 bis 50 C-Atomen mit einem Schmelzpunkt im Bereich von 50°C bis 110°C, die die Ölphase verdicken, sind ausgewählt aus C16-36-Alkylstearaten, insbesondere C₂₀-C₄₀-Alkylstearaten, C₁₈₋₃₈-Alkylhydroxystearoylstearaten, C₂₀₋₄₀-Alkylerucaten, Cetearylbehenat, Cetylbehenat, Stearylbehenat sowie Mischungen dieser Substanzen.

Erfindungsgemäß bevorzugte Triglyceride gesättigter und gegebenenfalls hydroxylierter C₁₂₋₃₀-Fettsäuren mit einem Schmelzpunkt im Bereich von 50°C bis 110°C, die die Ölphase verdicken, sind in erfindungsgemäß bevorzugten Blondierpasten in einer Gesamtmenge von 0,1 - 5 Gew.-%, bevorzugt 0,2 - 4 Gew.-%, besonders bevorzugt 0,3 - 2 Gew.-%, außerordentlich bevorzugt 0,4 - 1 Gew.-%, enthalten, jeweils bezogen auf das Gewicht der Blondierpaste.

Triglyceride im Sinne der vorliegenden Erfindung sind Triester des Glycerins, also Ester, bei denen alle OH-Gruppen des Glycerins mit Säure, vorliegend mit einer gesättigten und gegebenenfalls hydroxylierten C₁₂₋₃₀-Fettsäure, verestert sind.

Erfindungsgemäß bevorzugte Triglyceride gesättigter und gegebenenfalls hydroxylierter C₁₂₋₃₀-Fettsäuren mit einem Schmelzpunkt im Bereich von 50°C bis 110°C, die die Ölphase verdicken, sind ausgewählt aus gehärteten Triglyceridfetten, insbesondere hydriertem Palmöl, hydriertem Kokosöl, hydriertem Rizinusöl, Glyceryltribehenat (Tribehenin) und Glyceryltri-12-hydroxystearat, sowie Mischungen hiervon. Erfindungsgemäß besonders bevorzugt ist hydriertes Rizinusöl, erhältlich z.B. als Handelsprodukt Cutina® HR.

Erfindungsgemäß besonders bevorzugte Blondierpasten enthalten mindestens ein Triglycerid gesättigter und gegebenenfalls hydroxylierter C₁₂₋₃₀-Fettsäuren mit einem Schmelzpunkt im Bereich von 50°C bis 110°C, ausgewählt aus gehärteten Triglyceridfetten, insbesondere hydriertem Palmöl, hydriertem Kokosöl, hydriertem Rizinusöl, Glyceryltribehenat (Tribehenin) und Glyceryltri-12-hydroxystearat, sowie Mischungen hiervon, wobei hydriertes Rizinusöl besonders bevorzugt ist, in einer Gesamtmenge von 0,1 - 5 Gew.-%, bevorzugt 0,2 - 4 Gew.-%, besonders bevorzugt 0,3 - 2 Gew.-%, außerordentlich bevorzugt 0,4 - 1 Gew.-%, jeweils bezogen auf das Gewicht der Blondierpaste. Erfindungsgemäß besonders bevorzugte Blondierpasten enthalten weiterhin mindestens einen oder mehrere hydrophile Verdicker, der bevorzugt ausgewählt ist aus Polysacchariden, die chemisch und/oder physikalisch modifiziert sein können, Acrylsäurehomo- und -copolymeren, Methacrylsäurehomo- und -copolymeren, Itaconsäurehomo- und -copolymeren, und Mischungen dieser Polymere. Als hydrophile Verdicker sind insbesondere Verbindungen aus der Gruppe der Polysaccharide geeignet. Als Beispiele können Vertreter der Cellulosen (Cellulose selbst sowie ihre Derivate), der Alginsäuren (sowie ihrer entsprechenden physiologisch verträglichen Salze, der Alginate), Agar Agar (mit dem in Agar Agar als Hauptbestandteil vorhandenen Polysaccharid Agarose), Stärke-Fraktionen und Derivate wie Amylose, Amylopektin und Dextrine, Karaya-Gummi, Johannisbrotkernmehl, Gummi Arabicum, Dextrane, Guar Gum und Xanthan Gum genannt werden.

Geeignete Cellulose-Derivate sind Methylcellulosen, Ethylcellulosen, Hydroxyalkylcellulosen (wie beispielsweise Hydroxyethylcellulose), Methylhydroxyalkylcellulosen und Carboxymethylcellulosen (wie beispielsweise solche mit der INCI-Bezeichnung Cellulose Gum) sowie ihre physiologisch verträglichen Salze.

Bevorzugt werden aus der Gruppe der Polysaccharide für die Verdickung der erfindungsgemäßen Mittel anionische Polysaccharide wie Carboxymethylcellulosen, Alginsäuren und Xanthan Gum ausgewählt.

Carboxymethylcellulosen, Alginsäuren und Xanthan Gum werden neben ihren physiologisch verträglichen Salzen im Rahmen der vorliegenden Erfindung als anionische Polysaccharide bezeichnet, da die in diesen Polysacchariden vorhandenen Carbonsäuregruppierungen in Wasser bzw. wasserhaltiger Formulierung zwangsläufig in mehr oder weniger starkem Ausmaß dissoziieren, wodurch anionische Carboxylat-Gruppen gebildet werden, deren Anzahl mit steigendem pH-Wert weiter zunimmt.

In bevorzugten Ausführungsformen ist im Hinblick auf eine zuverlässige Viskositätseinstellung und rückstandsfreie Anwendung auf Keratinfasern und der Kopfhaut als hydrophiler Verdicker Carboxymethylcellulose (vorzugsweise Carboxymethylcellulose mit der INCI-Bezeichnung Cellulose Gum) enthalten. Carboxymethylcellulose kann in einer bevorzugten Ausführungsform als einziger hydrophiler Verdicker enthalten sein. Bevorzugt ist aber auch insbesondere eine Kombination von Carboxymethylcellulose und Xanthan (vorzugsweise Xanthan mit der INCI-Bezeichnung Xanthan Gum) bzw. von deren physiologisch verträglichen Salzen.

Zu den physiologisch verträglichen Salzen sind insbesondere die Natriumsalze, aber auch die Kaliumsalze, sowie weiterhin Magnesium- und Calciumsalze zu verstehen.

Erfindungsgemäß besonders bevorzugte Blondierpasten enthalten mindestens einen hydrophilen Verdicker in einer Gesamtmenge von 0,1 bis 5 Gew.-%, bevorzugt von 0,5 bis 4 Gew.-%, weiter bevorzugt von 1 bis 3 Gew.-% und ganz besonders bevorzugt von 1,7 bis 2,5 Gew.-%, jeweils bezogen auf das Gewicht der Blondierpaste, enthalten.

In einer weiteren bevorzugten Ausführungsform der vorliegenden Erfindung enthält die erfindungsgemäße Blondierpaste, jeweils bezogen auf ihr Gewicht, 0,1 bis 3 Gew.-%, bevorzugt 0,5 bis 2,5 Gew.-%, weiter bevorzugt 1 bis 2 Gew.-% , noch bevorzugter 1 bis 1,5 Gew.-%, Carboxymethylcellulose.

In einer weiteren bevorzugten Ausführungsform der vorliegenden Erfindung enthält die erfindungsgemäße Blondierpaste, jeweils bezogen auf ihr Gewicht, 0,1 bis 3 Gew.-%, bevorzugt 0,5 bis 2,5 Gew.-%, weiter bevorzugt 1 bis 2 Gew.-% , noch bevorzugter 1 bis 1,5 Gew.-%, Xanthan.

Erfindungsgemäß als hydrophile Verdicker geeignete Acrylsäurehomo- und -copolymere, Methacrylsäurehomo- und -copolymere, Itaconsäurehomo- und -copolymere sind bevorzugt ausgewählt aus der Gruppe, die gebildet wird von den vernetzten und unvernetzten Homo- oder Copolymeren der Acrylsäure, der Methacrylsäure sowie deren Salzen und Alkylestern, Homo- oder Copolymeren von Acrylsäureamiden und/oder Methacrylsäureamiden, Copolymeren aus Acrylsäure und Acrylsäureamiden sowie deren Mischungen, Copolymeren der ethoxylierten C1-C6-Alkylester der Methacrylsäure und der sulfonierten Acrylsäureamide sowie deren Salzen und vernetzten Copolymeren der Methacrylsäure, der Acrylsäureamide und der sulfonierten Acrylsäureamide sowie deren Salzen. Die vorstehend genannten Polymere und Copolymere können vernetzt oder unvernetzt sein. Sofern die vorstehend genannten Polymere und Copolymere keine Alkylgruppen mit einer Kettenlänge von mindestens 8 Kohlenstoffatomen aufweisen, sind sie bevorzugt vernetzt. Sofern die vorstehend genannten Polymere und Copolymere Alkylgruppen mit einer Kettenlänge von mindestens 8 Kohlenstoffatomen aufweisen, sind sie bevorzugt unvernetzt.

Beispiele für als hydrophile Verdicker bevorzugte Polymere sind beispielsweise das unter der INCI-Bezeichnung bekannte Copolymer Ammonium Acryloyldimethyltaurate / Beheneth-25 methacrylate Crosspolymer (Handelsbezeichnung: Aristoflex HMB; Clariant), die unter der INCI-Bezeichnung Acrylates/C10-30 Alkyl Acrylate Crosspolymer bekannten Copolymere sowie das unter der INCI-Bezeichnung Polyacrylate Crosspolymer-11 bekannte vernetzte Copolymer (Handelsbezeichnung: Aristoflex Velvet; Clariant).

Weiterhin enthalten erfindungsgemäß bevorzugte Blondierpasten einen oder mehrere alkoxylierte Fettalkohole, insbesondere ethoxylierte Fettalkohole. Geeignet sind insbesondere Fettalkohole mit 12 bis 80 Ethylenoxidgruppen, bevorzugt 25 bis 50 Ethylenoxidgruppen. Als ethoxylierte Fettalkohole sind erfindungsgemäß solche der folgenden Formel (FAEO) geeignet:

RO[CH₂CH₂-O]ₙH (FAEO)

worin R für eine unverzweigte oder verzweigte, gesättigte oder ungesättigte C10-C24-Alkylgruppe steht und n für eine ganze Zahl von 12 bis 80 steht. Bevorzugt steht R für eine unverzweigte, gesättigte C12-C18-Alkylgruppe oder für eine unverzweigte, einfach ungesättigte C12-C18-Alkylgruppe. In Formel (FAEO) steht n bevorzugt für eine ganze Zahl von 20 bis 60, besonders bevorzugt steht n für eine ganze Zahl von 25 bis 50. Beispiele für alkoxylierte Fettalkohole der Formel (FAEO) sind Laureth-20, Laureth-25, Laureth-30, Laureth-40, Laureth-50, Myreth- 20, Myreth-25, Myreth-30, Myreth-40, Myreth-50, Ceteth-20, Ceteth-25, Ceteth-30, Ceteth-40, Ceteth-50, Steareth-20, Steareth- 25, Steareth-30, Steareth-40, Steareth-50, Ceteareth-20, Ceteareth-25, Ceteareth-30, Ceteareth-40, Ceteareth-50, Oleth-20, Oleth-25, Oleth-30, Oleth-40 und Oleth-50.

Erfindungsgemäß besonders bevorzugte Blondierpasten enthalten einen oder mehrere alkoxylierte Fettalkohole, insbesondere ethoxylierte Fettalkohole der vorstehenden Formel (FAEO) in einer Gesamtmenge von 0,2 bis 13,0 Gew.-%, bevorzugt 2,0 bis 8,0 Gew.-%, weiter bevorzugt von 3,0 bis 7,0 Gew.-%, jeweils bezogen auf das Gewicht der erfindungsgemäßen Blondierpaste.

Erfindungsgemäß besonders bevorzugte Blondierpasten enthalten Ceteareth-30 und/oder Ceteareth-50 oder eine Kombination davon. Insbesondere ist eine Kombination von Ceteareth-30 und Ceteareth-50 bevorzugt. Dabei ist Ceteareth-30 bevorzugt in einer Menge von 0,1 bis 5 Gew.- % enthalten, weiter bevorzugt 0,2 bis 1 Gew.-%, noch bevorzugter 0,3 bis 0,7 Gew.-%, und Ceteareth-50 in einer Menge von 0,1 bis 8 Gew.-%, weiter bevorzugt 2 bis 6 Gew.-%, noch bevorzugter 3 bis 5 Gew.-%, jeweils bezogen auf das Gewicht der erfindungsgemäßen Blondierpaste. Es hat sich gezeigt, dass bei einer Menge von Ceteareth-30 und Ceteareth-50 in den genannten Bereichen, insbesondere bei einer Kombination von Ceteareth-30 und Ceteareth-50, besonders vorteilhafte Eigenschaften der erfindungsgemäß besonders bevorzugten Blondierpasten und der daraus angefertigten Anwendungsmischungen erzielt werden.

Ein weiterer Gegenstand der vorliegenden Erfindung ist ein Verfahren zur Aufhellung von keratinischen Fasern, insbesondere menschlichen Haaren, bei dem eine erfindungsgemäße oder erfindungsgemäß bevorzugte Blondierpaste mit einer Oxidationszusammensetzung vermischt wird, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei die Blondierpaste (B) und die Oxidationszusammensetzung (Ox) bevorzugt in einem gewichtsbezogenen Verhältnis (B) : (Ox) von 0,2 - 1, besonders bevorzugt 0,3 - 0,8, weiter bevorzugt 0,4 - 0,7, außerordentlich bevorzugt 0,5 - 0,6, miteinander vermischt werden.

Die im erfindungsgemäßen Aufhellverfahren eingesetzte Oxidationszusammensetzung (Ox) enthält im Wesentlichen Wasser und Wasserstoffperoxid. Die Konzentration des Wasserstoffperoxids wird einerseits von den gesetzlichen Vorgaben und andererseits von dem gewünschten Effekt bestimmt. Sie beträgt 0,5 - 20 Gew.-%, bevorzugt 3-12 Gew.-%, besonders bevorzugt 6-9 Gew.- % Wasserstoffperoxid (berechnet als 100%iges H₂O₂), jeweils bezogen auf das Gewicht der Oxidationszusammensetzung (Ox).

Die Oxidationszusammensetzung (Ox) besitzt zur Stabilisierung des Wasserstoffperoxids bevorzugt einen sauren pH-Wert, insbesondere einen pH-Wert im Bereich von 2,5 bis 5,5, gemessen bei 20°C. Zur Stabilisierung des Wasserstoffperoxids sind weiterhin bevorzugt Komplexbildner, Konservierungsmittel und/oder Puffersubstanzen enthalten.

Erfindungsgemäß bevorzugt ist die Blondierpaste so zusammengesetzt, dass die Mischung mit der vorgenannten Oxidationszusammensetzung (Ox), also das anwendungsbereite Farbveränderungsmittel, insbesondere Blondiermittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist, jeweils gemessen bei 20 °C.

Erfindungsgemäß besonders bevorzugt verwendete Oxidationszusammensetzungen (Ox) enthalten weiterhin mindestens ein Öl und/oder mindestens eine Fettkomponente mit einem Schmelzpunkt im Bereich von 23 - 110 °C, bevorzugt in einer Gesamtmenge von 0,1 - 60 Gew.-%, besonders bevorzugt 0,5 - 40 Gew.-%, außerordentlich bevorzugt 2 - 24 Gew.-%, jeweils bezogen auf das Gewicht der erfindungsgemäß bevorzugt verwendeten Oxidationszusammensetzung (Ox). Die für die erfindungsgemäß bevorzugt verwendeten Oxidationszusammensetzungen (Ox) geeigneten Öle sind die gleichen Öle, die vorstehend als geeignetes Trägermedium für die Blondierpasten offenbart sind.

In den Oxidationszusammensetzungen (Ox) erfindungsgemäß bevorzugt verwendete Fettkomponenten mit einem Schmelzpunkt im Bereich von 23 - 110 °C sind ausgewählt aus linearen gesättigten 1-Alkanolen mit 12-30 Kohlenstoffatomen, bevorzugt in einer Gesamtmenge von 0,1 - 8 Gew.-%, besonders bevorzugt 2,0 bis 6,0 Gew.-%, jeweils bezogen auf das Gewicht der erfindungsgemäß verwendeten Oxidationszusammensetzung (Ox).

Bevorzugt ist das mindestens eine lineare gesättigte 1-Alkanol mit 12-30 Kohlenstoffatomen ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/ Stearylalkohol-Mischungen.

Erfindungsgemäß bevorzugt verwendete Oxidationszusammensetzungen (Ox) enthalten weiterhin, jeweils bezogen auf ihr Gewicht, mindestens ein lineares gesättigtes 1-Alkanol mit 12 - 30 Kohlenstoffatomen in einer Gesamtmenge von 0,1 - 8 Gew.-%, bevorzugt in einer Gesamtmenge von 2 - 6 Gew.-%, wobei mindestens ein 1-Alkanol, ausgewählt aus Cetylalkohol, Stearylalkohol und Cetylalkohol/Stearylalkohol-Mischungen, enthalten ist.

Weitere erfindungsgemäß bevorzugt verwendete Oxidationszusammensetzungen (Ox) enthalten mindestens eine Fettkomponente mit einem Schmelzpunkt im Bereich von 23 - 110 °C, die ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂-C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen.

Weitere erfindungsgemäß bevorzugt verwendete Oxidationszusammensetzungen (Ox) enthalten mindestens ein Tensid oder mindestens einen Emulgator, bevorzugt in einer Gesamtmenge von 0,5 - 10 Gew.-%, bevorzugt 1 - 5 Gew.-%, jeweils bezogen auf das Gewicht der erfindungsgemäß verwendeten Oxidationszusammensetzung (Ox).

Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann. Besonders bevorzugt ist diese C₈-C₂₈-Alkylkette linear. Basiseigenschaften der Tenside und Emulgatoren sind die orientierte Absorption an Grenzflächen sowie die Aggregation zu Mizellen und die Ausbildung von lyotropen Phasen.

Erfindungsgemäß sind anionische, nichtionische und kationische Tenside besonders geeignet. Aber auch zwitterionische und amphotere Tenside sind erfindungsgemäß sehr geeignet.

Als anionische Tenside eignen sich in den erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind lineare und verzweigte Fettsäuren mit 8 bis 30 C-Atomen (Seifen), Alkylethercarbonsäuren, Acylsarcoside, Acyltauride, Acylisethionate, Sulfobernsteinsäuremono-, -dialkylester und Sulfobernsteinsäuremono-alkylpolyoxyethylester, lineare Alkansulfonate, lineare alpha-Olefinsulfonate, Alkylsulfate und Alkylethersulfate sowie Alkyl- und/oder Alkenylphosphate. Bevorzugte anionische Tenside sind Alkylsulfate, Alkylethersulfate und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen im Molekül. Beispiele solcher Tenside sind die Verbindungen mit den INCI-Bezeichnungen Sodium Laureth Sulfate, Sodium Lauryl Sulfate, Sodium Myreth Sulfate oder Sodium Laureth Carboxylate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 4 bis 50 Mol Ethylenoxid und/oder 0 bis 5 Mol Propylenoxid an lineare und verzweigte Fettalkohole, an Fettsäuren und an Alkylphenole, jeweils mit 8 bis 20 C-Atomen in der Alkylgruppe, ethoxylierte Mono-, Di- und Triglyceride, wie beispielsweise Glycerinmonolaurat + 20 Ethylenoxid, und Glycerinmonostearat + 20 Ethylenoxid, Sorbitanfettsäureester und Anlagerungeprodukte von Ethylenoxid an Sorbitanfettsäureester wie beispielsweise die Polysorbate (Tween 20, Tween 21, Tween 60, Tween 61, Tween 81), Anlagerungsprodukte von Ethylenoxid an Fettsäurealkanolamide und Fettamine, sowie Alkylpolyglycoside. Als nichtionische Tenside eignen sich insbesondere C₈-C₂₂-Alkylmono-und -oligoglycoside und deren ethoxylierte Analoga sowie Ethylenoxid-Anlagerungsprodukte an gesättigte oder ungesättigte lineare Fettalkohole mit jeweils 2 bis 30 Mol Ethylenoxid pro Mol Fettalkohol.

Weitere erfindungsgemäß bevorzugt verwendete Oxidationszusammensetzungen sind dadurch gekennzeichnet, dass das mindestens eine anionische Tensid ausgewählt ist aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül.

Weitere erfindungsgemäß bevorzugt verwendete Oxidationszusammensetzungen sind dadurch gekennzeichnet, dass mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen, im Molekül, enthalten ist, wobei besonders bevorzugt das Gewichtsverhältnis der Gesamtheit aller anionischen Tenside zur Gesamtheit aller nichtionischen Tenside im Bereich von 5 - 50, bevorzugt 10-30 liegt.

Als kationische Tenside eignen sich in erfindungsgemäß bevorzugt verwendeten Oxidationszusammensetzungen (Ox) prinzipiell alle für die Verwendung am menschlichen Körper geeigneten kationischen oberflächenaktiven Stoffe. Diese sind gekennzeichnet durch mindestens eine wasserlöslich machende, kationische Gruppe, wie z. B. eine quaternäre Ammonium-Gruppe, oder durch mindestens eine wasserlöslich machende, kationisierbare Gruppe, wie z. B. eine Amin-Gruppe, und weiterhin mindestens eine (lipophil wirkende) Alkylgruppe mit 6 bis 30 C-Atomen oder mindestens eine (lipophil wirkende) Imidazol-Gruppe oder mindestens eine (lipophil wirkende) Imidazylalkyl-Gruppe.

Erfindungsgemäß besonders bevorzugt verwendete Oxidationszusammensetzungen (Ox) enthalten mindestens ein kationisches Tensid, das bevorzugt ausgewählt ist aus quarternären Ammoniumverbindungen mit mindestens einem C8-C24-Alkylrest, Esterquats und Amidoaminen mit jeweils mindestens einem C8-C24-Acylrest sowie Mischungen hiervon. Bevorzugte quaternäre Ammoniumverbindungen mit mindestens einem C8-C24-Alkylrest sind Ammoniumhalogenide, insbesondere Chloride, und Ammoniumalkylsulfate, wie Methosulfate oder Ethosulfate, wie C8-C24-Alkyltrimethylammoniumchloride, C8-C24-Dialkyldimethylammoniumchloride und C8-C24-Trialkyl-methylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83, Quaternium-87 und Quaternium-91 bekannten Imidazolium-Verbindungen. Die Alkylketten der oben genannten Tenside weisen bevorzugt 8 bis 24 Kohlenstoffatome auf. Bei Esterquats handelt es sich um kationische Tenside, die sowohl mindestens eine Esterfunktion als auch mindestens eine quartäre Ammoniumgruppe als Strukturelement und weiterhin mindestens eine C8-C24-Alkylrest oder C8-C24-Acylrest enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. N,N-Bis(2-Palmitoyloxyethyl)dimethylammoniumchlorid, Distearoylethyl Dimonium Methosulfate und Distearoylethyl Hydroxyethylmonium Methosulfate sind bevorzugte Beispiele für solche Esterquats.

Die Alkylamidoamine werden üblicherweise durch Amidierung natürlicher oder synthetischer C8-C24-Fettsäuren und Fettsäureschnitte mit Di-(C1-C3)alkylaminoaminen hergestellt. Eine erfindungsgemäß besonders geeignete Verbindung aus dieser Substanzgruppe ist Stearamidopropyldimethylamin.

Erfindungsgemäß besonders bevorzugt verwendete Oxidationszusammensetzungen (Ox) enthalten mindestens ein kationisches Tensid in einer Gesamtmenge von 0,01 - 5 Gew.-%, bevorzugt 0,1 - 3 Gew.-%, besonders bevorzugt 0,3 - 2 Gew.-%, jeweils bezogen auf das Gewicht der erfindungsgemäß verwendeten Oxidationszusammensetzung (Ox).

Ein weiterer Gegenstand der vorliegenden Erfindung ist eine Mehrkomponentenverpackungseinheit (Kit-of-Parts) zur Aufhellung von keratinischen Fasern, die mindestens zwei getrennt voneinander verpackte Komponenten enthält und die dadurch gekennzeichnet ist, dass
i) die erste Komponente (I) eine erfindungsgemäße oder erfindungsgemäß bevorzugte Blondierpaste ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei die Komponenten (I) und (II) bevorzugt in einem gewichtsbezogenen Verhältnis (I) : (II) von 0,2 - 1, besonders bevorzugt 0,3 - 0,8, weiter bevorzugt 0,4 - 0,7, außerordentlich bevorzugt 0,5 - 0,6, zueinander vorliegen.

Ein weiterer Gegenstand der vorliegenden Erfindung ist eine Mehrkomponentenverpackungseinheit (Kit-of-Parts) zur Farbveränderung von keratinischen Fasern, insbesondere menschlichen Haaren, enthaltend mindestens drei getrennt voneinander verpackte Komponenten, wobei
i) die erste Komponente (I) eine erfindungsgemäße oder erfindungsgemäß bevorzugte Blondierpaste ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
iii) die dritte Komponente (III) eine Alkalisierungszusammensetzung (Alk) ist, die Wasser und mindestens ein Alkalisierungsmittel, das ausgewählt ist aus Ammoniak, Alkanolaminen und Mischungen hiervon, enthält und einen pH-Wert im Bereich von 8 - 12, bevorzugt von 9-11, besonders bevorzugt von 9,5 - 10,5, aufweist, jeweils gemessen bei 20°C,
wobei die Blondierpaste (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3 - 2,7) : (2,3 - 2,7), außerordentlich bevorzugt 1:2:2, zueinander vorliegen.

Ein weiterer Gegenstand der vorliegenden Erfindung ist eine Mehrkomponentenverpackungseinheit (Kit-of-Parts) zur Farbveränderung von keratinischen Fasern, insbesondere menschlichen Haaren, enthaltend mindestens drei getrennt voneinander verpackte Komponenten, wobei
i) die erste Komponente (I) eine erfindungsgemäße oder erfindungsgemäß bevorzugte Blondierpaste ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
iii) die dritte Komponente (III) eine Alkalisierungszusammensetzung (Alk) ist, die Wasser und mindestens ein Alkalisierungsmittel, das ausgewählt ist aus Ammoniak, Alkanolaminen und Mischungen hiervon, enthält und einen pH-Wert im Bereich von 8 - 12, bevorzugt von 9-11, besonders bevorzugt von 9,5 - 10,5, aufweist, jeweils gemessen bei 20°C,
wobei die Blondierpaste (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3 - 2,7) : (2,3 - 2,7), außerordentlich bevorzugt 1:2:2, zueinander vorliegen.

Eine Mehrkomponentenverpackungseinheit umfasst mehrere Einzelkomponenten, die getrennt voneinander konfektioniert sind, sowie eine gemeinsame Verpackung für diese Komponenten, zum Beispiel eine Faltschachtel. Darin werden die Komponenten jeweils getrennt in verschiedenen Containern bereitgestellt. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder einer ähnlichen Umhüllung vorliegt. Dem Umhüllungsmaterial sind erfindungsgemäß keine Grenzen gesetzt. Bevorzugt handelt es sich jedoch dabei um Umhüllungen aus Kunststoff.

Darüber hinaus kann die Verpackungseinheit Applikationshilfen, wie Kämme, Bürsten oder Pinsel, persönliche Schutzkleidung, insbesondere Einweg-Handschuhe, sowie eine Gebrauchsanleitung umfassen.

In einer weiteren bevorzugten Ausführungsform der Erfindung kann eine erfindungsgemäße oder erfindungsgemäß bevorzugte Blondierpaste mit einer Alkalisierungszusammensetzung und mit einer Oxidationszusammensetzung zu einem aufhellenden Farbveränderungsmittel für keratinische Fasern kombiniert werden.

Da bei der Behandlung von keratinischer Fasern, insbesondere Haaren, mit Oxidationsmitteln, insbesondere mit Wasserstoffperoxid, der fasereigene Farbstoff Melanin zu einem gewissen Grad zerstört wird, werden die Fasern/Haare zwangsläufig aufgehellt, ändern also ihre Farbe auch ohne die Gegenwart eines Farbstoffs. Daher umfasst der Begriff "Farbveränderung" im Sinne der vorliegenden Anmeldung sowohl die Aufhellung als auch die Färbung mit einem oder mehreren Farbstoffen.

Die erfindungsgemäß verwendete Alkalisierungszusammensetzung (Alk) enthält Wasser und mindestens ein Alkalisierungsmittel, das ausgewählt ist aus Ammoniak, Alkanolaminen und Mischungen hiervon, und weist einen pH-Wert im Bereich von 8 - 12, bevorzugt von 9-11, besonders bevorzugt von 9,5 - 10,5, auf, jeweils gemessen bei 20°C. Bevorzugte Alkanolamine sind ausgewählt aus Monoethanolamin, 2-Amino-2-methylpropanol und Triethanolamin sowie Mischungen hiervon, wobei Monoethanolamin besonders bevorzugt ist. Ein außerordentlich bevorzugtes Alkalisierungsmittel ist Ammoniak.

Üblicherweise wird Ammoniak (NH₃) in Form seiner wässrigen Lösung eingesetzt. Wässrige Ammoniak-Lösungen enthalten Ammoniak (NH₃) oft in Konzentrationen von 10 bis 32 Gew.-%. Bevorzugt ist hierbei der Einsatz einer wässrigen Ammoniak-Lösung, die 25 Gew.-% Ammoniak (NH₃) enthält.

Neben Ammoniak und Alkanolaminen kann mindestens ein weiteres Alkalisierungsmittel enthalten sein, das ausgewählt ist aus Alkalimetallsilikaten, Erdalkalimetallsilikaten, Erdalkalimetallhydroxidcarbonaten, Erdalkalimetallcarbonaten, Alkalimetallmetasilikaten, Erdalkalimetallmetasilikaten, Alkalimetallhydroxiden und Erdalkalimetallhydroxiden, sowie Mischungen dieser Substanzen. Bevorzugt sind Ammoniak und/oder Monoethanolamin in den erfindungsgemäß bevorzugt verwendeten Alkalisierungszusammensetzungen in Mengen von 0,01 - 10 Gew.-%, bevorzugt von 0,1 - 7,5 Gew.-%, weiter bevorzugt von 0,5 - 5,5 Gew.-% und besonders bevorzugt von 1,5 - 4,5 Gew.- % - jeweils bezogen auf das Gewicht der Alkalisierungszusammensetzung - enthalten.

Ein weiterer Gegenstand der vorliegenden Erfindung ist ein Verfahren zur Farbveränderung von keratinischen Fasern, insbesondere menschlichen Haaren, bei dem eine erfindungsgemäße oder erfindungsgemäß bevorzugte Blondierpaste mit einer Oxidationszusammensetzung (Ox), die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist,
und zusätzlich mit einer Alkalisierungszusammensetzung (Alk), die Wasser und mindestens ein Alkalisierungsmittel, das ausgewählt ist aus Ammoniak, Alkanolaminen und Mischungen hiervon, enthält und einen pH-Wert im Bereich von 8-12, bevorzugt von 9-11, besonders bevorzugt von 9,5 - 10,5, aufweist, jeweils gemessen bei 20°C,
vermischt wird,
unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei die Blondierpaste (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3-2,7) : (2,3 - 2,7), außerordentlich bevorzugt 1:2:2, miteinander vermischt werden.

Erfindungsgemäß bevorzugt ist die Blondierpaste so zusammengesetzt, dass die Mischung mit der vorgenannten Oxidationszusammensetzung (Ox) und mit der vorgenannten Alkalisierungszusammensetzung (Alk), also das anwendungsbereite Farbveränderungsmittel, insbesondere Blondiermittel, einen alkalischen pH-Wert, bevorzugt einen pH-Wert von 8 bis 11,5, besonders bevorzugt einen pH-Wert von 8,5 bis 11, außerordentlich bevorzugt einen pH-Wert von 9,0 bis 10,5, aufweist, jeweils gemessen bei 20 °C.

Die anwendungsbereiten Mischungen aus einer erfindungsgemäßen oder erfindungsgemäß bevorzugten Blondierpaste mit einer der vorgenannten Oxidationszusammensetzungen (Ox) und ggf. mit einer der vorgenannten Alkalisierungszusammensetzungen (Alk) weisen bevorzugt eine Viskosität im Bereich von 3000 bis 20000 mPas, besonders bevorzugt 6000 bis 15000 mPas, auf, jeweils gemessen bei 20°C mit einem Haake-Zylinder/Zylinder-Viskosimeter, Dreh-/Messsystem SV I mit einer Kühlzeit von 5 Minuten. Bei diesem Messverfahren wird der Viskositätswert bei einer Scherrate von 1/7,2s ermittelt. Das Messprogramm arbeitet mit der Rampe von 0-1/60s. Eine Viskosität in diesem Bereich erlaubt, dass das anwendungsbereite Mittel sich einerseits gut auftragen lässt und andererseits über ein solches Fließverhalten verfügt, dass es für das Mittel eine ausreichend lange Einwirkzeit am Wirkort auf den keratinischen Fasern garantiert.

Um die Mischbarkeit der erfindungsgemäß verwendeten Alkalisierungszusammensetzung mit der erfindungsgemäßen oder erfindungsgemäß bevorzugten Blondierpaste und der erfindungsgemäß verwendeten Oxidationszusammensetzung zu erleichtern sowie die Anwendungseigenschaften der resultierenden Anwendungsmischung zu verbessern, enthält die erfindungsgemäß bevorzugt verwendete Alkalisierungszusammensetzung bevorzugt, jeweils bezogen auf ihr Gewicht, mindestens ein Tensid in einer Gesamtmenge von 0,5 - 10 Gew.-%, bevorzugt 2-8 Gew.-%.

Die für die erfindungsgemäß bevorzugt verwendeten Alkalisierungszusammensetzungen (Alk) geeigneten Tenside sind ausgewählt aus den gleichen anionischen, kationischen, nichtionischen, amphoteren und zwitterionischen Tensiden und Emulgatoren, die vorstehend als für die bevorzugt verwendeten Oxidationszusammensetzungen (Ox) geeignete Tenside und Emulgatoren offenbart sind.

Erfindungsgemäß besonders bevorzugt verwendete Alkalisierungszusammensetzungen (Alk) enthalten weiterhin mindestens ein Öl und/oder mindestens eine Fettkomponente mit einem Schmelzpunkt im Bereich von 23 - 110 °C, bevorzugt in einer Gesamtmenge von 0,1 - 60 Gew.-%, besonders bevorzugt 0,5 - 40 Gew.-%, außerordentlich bevorzugt 2 - 24 Gew.-%, jeweils bezogen auf das Gewicht der erfindungsgemäß bevorzugt verwendeten Alkalisierungszusammensetzung (Alk). Die für die erfindungsgemäß bevorzugt verwendeten Alkalisierungszusammensetzungen (Alk) geeigneten Öle sind die gleichen Öle, die vorstehend als geeignetes Trägermedium für die Blondierpaste offenbart sind.

In den Alkalisierungszusammensetzungen (Alk) erfindungsgemäß bevorzugt verwendete Fettkomponenten mit einem Schmelzpunkt im Bereich von 23 - 110 °C sind ausgewählt aus linearen gesättigten 1-Alkanolen mit 12-30 Kohlenstoffatomen, bevorzugt in einer Gesamtmenge von 0,1 - 20 Gew.-%, besonders bevorzugt 3-15 Gew.-%, außerordentlich bevorzugt 5-10 Gew.-%, jeweils bezogen auf das Gewicht der erfindungsgemäß verwendeten Alkalisierungszusammensetzung. Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/ Stearylalkohol-Mischungen.

Erfindungsgemäß bevorzugt verwendete Alkalisierungszusammensetzungen (Alk) enthalten weiterhin, jeweils bezogen auf ihr Gewicht, mindestens ein lineares gesättigtes 1-Alkanol mit 12 - 30 Kohlenstoffatomen in einer Gesamtmenge von 0,1 - 20 Gew.-%, bevorzugt in einer Gesamtmenge von 3 - 15Gew.-%, außerordentlich bevorzugt 5 - 10 Gew.-%, wobei mindestens ein 1-Alkanol, ausgewählt aus Cetylalkohol, Stearylalkohol und Cetylalkohol/Stearylalkohol-Mischungen, enthalten ist.

Weitere erfindungsgemäß bevorzugt verwendete Alkalisierungszusammensetzungen (Alk) enthalten mindestens eine Fettkomponente mit einem Schmelzpunkt im Bereich von 23 - 110 °C, die ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄-C₃₆-Carbonsäuren sowie Mischungen der vorgenannten Substanzen.

Weiterhin können die erfindungsgemäßen oder erfindungsgemäß bevorzugten Blondierpasten und/ oder die erfindungsgemäß bevorzugt verwendeten Alkalisierungszusammensetzungen mindestens einen direktziehenden Farbstoff enthalten. Dabei handelt es sich um Farbstoffe, die direkt auf das Haar aufziehen und keinen oxidativen Prozess zur Ausbildung der Farbe benötigen. Zur Mattierung von durch Melaninabbauprodukte hervorgerufene unerwünschte Restfarbeindrücke, insbesondere im rötlichen oder bläulichen Bereich, sind besonders bevorzugt bestimmte direktziehende Farbstoffe der komplementären Farben enthalten. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe sind jeweils bevorzugt in einer Menge von 0,001 bis 2 Gew.-%, bezogen auf das Gewicht der Blondierpaste oder der Alkalisierungszusammensetzung (Alk), eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterozyklus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß ganz besonders bevorzugt ist eine Kombination aus Tetrabromphenolblau und Acid Red 92 enthalten.

Als weiteren optionalen Inhaltsstoff enthält die erfindungsgemäß bevorzugt verwendete Alkalisierungszusammensetzung mindestens ein Oxidationsfarbstoffvorprodukt, das vorzugsweise aus einer oder mehreren Entwicklerkomponenten und gegebenenfalls einer oder mehreren Kupplerkomponenten ausgewählt ist.

Besonders bevorzugt ist mindestens ein Oxidationsfarbstoffvorprodukt in einer Gesamtmenge von 0,0001 bis 10,0 Gew.-%, bevorzugt 0,001 bis 8 Gew.-%, jeweils bezogen auf das Gewicht der erfindungsgemäß bevorzugt verwendeten Alkalisierungszusammensetzung, enthalten.

Es kann erfindungsgemäß bevorzugt sein, als Entwicklerkomponente mindestens eine Verbindung aus der Gruppe auszuwählen, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethyl-aminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Bevorzugt ist mindestens eine Entwicklerkomponente in einer Gesamtmenge von 0,0001 bis 10,0 Gew.-%, bevorzugt 0,001 bis 8 Gew.-%, jeweils bezogen auf das Gewicht der erfindungsgemäß bevorzugt verwendeten Alkalisierungszusammensetzung, enthalten.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Erfindungsgemäß bevorzugte Kupplerkomponenten sind ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Bevorzugt ist mindestens eine Kupplerkomponente in einer Gesamtmenge von 0,0001 bis 10,0 Gew.-%, bevorzugt 0,001 bis 8 Gew.-%, jeweils bezogen auf das Gewicht der erfindungsgemäß bevorzugt verwendeten Alkalisierungszusammensetzung, enthalten.

Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

Bevorzugt beträgt die Einwirkzeit 5 bis 60 min, insbesondere 5 bis 50 min, besonders bevorzugt 10 bis 45 min. Während der Einwirkzeit der Mittel auf der Faser kann es vorteilhaft sein, den Aufhell- oder Farbveränderungsvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Mittel ergeben bereits bei physiologisch verträglichen Temperaturen von unter 45°C gute Behandlungsergebnisse.

Nach Ende des Farbveränderungsvorgangs werden alle auf den Keratinfasern befindlichen Komponenten mit Wasser oder einem tensidhaltigen Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Farbveränderungsmittel einen höheren Tensidgehalt aufweist.

Ein weiterer Gegenstand der vorliegenden Erfindung ist die Verwendung von Bernsteinsäure und/oder mindestens einem Salz der Bernsteinsäure,
in Kombination mit
Arginin und Lysin oder mindestens einem der Salze dieser Aminosäuren,
in einer Blondierpaste, die mindestens ein Oxidationsmittel, ausgewählt aus Natriumpercarbonaten und anorganischen Salzen einer Peroxoschwefelsäure, und sowie Mischungen hiervon, und weiterhin 0 bis 4 Gew.-% Wasser, bezogen auf das Gewicht der Blondierpaste, enthält,
zur Verringerung der Schädigung an keratinischen Fasern, insbesondere menschlichen Haaren, die bewirkt wird durch die Behandlung dieser Fasern mit einer Mischung aus der Blondierpaste und einer Oxidationszusammensetzung, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist.

Das zu den erfindungsgemäßen und erfindungsgemäß bevorzugten Blondierpasten Gesagte gilt mutatis mutandis auch für die erfindungsgemäßen und erfindungsgemäß bevorzugten Mehrkomponentenverpackungseinheiten (Kits of parts).
Das zu den erfindungsgemäßen und erfindungsgemäß bevorzugten Blondierpasten Gesagte gilt mutatis mutandis auch für die erfindungsgemäßen und erfindungsgemäß bevorzugten Verfahren zur Aufhellung und/oder zur Farbveränderung der keratinischen Fasern.
Das zu den erfindungsgemäß und erfindungsgemäß bevorzugt verwendeten Oxidationszusammensetzungen oder Alkalisierungszusammensetzungen Gesagte gilt mutatis mutandis auch für die erfindungsgemäßen und erfindungsgemäß bevorzugten Mehrkomponentenverpackungseinheiten (Kits of parts).
Das zu den erfindungsgemäß und erfindungsgemäß bevorzugt verwendeten Oxidationszusammensetzungen oder Alkalisierungszusammensetzungen Gesagte gilt mutatis mutandis auch für die erfindungsgemäßen und erfindungsgemäß bevorzugten Verfahren zur Aufhellung und/oder zur Farbveränderung der keratinischen Fasern.

Das zu den erfindungsgemäßen und erfindungsgemäß bevorzugten Blondierpasten Gesagte gilt mutatis mutandis auch für die erfindungsgemäße Verwendung.
Das zu den erfindungsgemäß und erfindungsgemäß bevorzugt verwendeten Oxidationszusammensetzungen oder Alkalisierungszusammensetzungen Gesagte gilt mutatis mutandis auch für die erfindungsgemäße Verwendung.

### BEISPIELE

### 1. Erfindungsgemäße Blondierpasten-Formulierungen

### (soweit nicht anders angegeben, entsprechen die Mengenangaben Gewichts-%)

| | Nr. 1 | Nr. 2 | Nr. 3 | Nr. 4 | Nr. 5 | Nr. 6 |
|---|---|---|---|---|---|---|
| Paraffinum Liquidum (Mineralöl) (Visk. >7 mm²/s <20.5 mm²/s bei 20 °C) | 37 | 37 | 37 | 37 | 37 | 37 |
| Ethylene/Propylene/Styrene Copolymer | 0,2 | 0,2 | 0,2 | 0,2 | 0,2 | 0,2 |
| Butylene/Ethylene/Styrene Copolymer | 0,2 | 0,2 | 0,2 | 0,2 | 0,2 | 0,2 |
| Natriumcetearylsulfat | 0,5 | 0,5 | 0,5 | 0,5 | 0,5 | 0,5 |
| Cetearylalkohol | 5 | 5 | 5 | 5 | 5 | 5 |
| Ceteareth-50 | 4 | 4 | 4 | 4 | 4 | 4 |
| Castorwachs (Hydrogenated Castor Oil) | 0,5 | 0,5 | 0,5 | 0,5 | 0,5 | 0,5 |
| Ceteareth-30 | 0,5 | 0,5 | 0,5 | 0,5 | 0,5 | 0,5 |
| Xanthan Gum | 1,2 | 1,2 | 1,2 | 1,2 | 1,2 | 1,2 |
| Cellulose Gum | 1,2 | 1,2 | 1,2 | 1,2 | 1,2 | 1,2 |
| Natriummetasilikat | 6,5 | 6,5 | 6,5 | 6,5 | 6,5 | 6,5 |
| Kaliumpersulfat | 41,6 | 41,6 | 41,6 | 41,6 | 41,6 | 41,6 |
| Silica | 0,2 | 0,2 | 0,2 | 0,2 | 0,2 | 0,2 |
| Kaliumsulfat | 0,2 | 0,2 | 0,2 | 0,2 | 0,2 | 0,2 |
| Bernsteinsäure | 0,2 | 0,2 | 0,2 | - | - | - |
| Äpfelsäure | - | - | - | 0,2 | 0,2 | 0,2 |
| L-Arginin | 0,2 | 0,4 | - | 0,2 | 0,4 | - |
| L-Lysin | 0,2 | - | 0,4 | 0,2 | - | 0,4 |
| Parfüm | 0,6 | 0,6 | 0,6 | 0,6 | 0,6 | 0,6 |

### 1.2 Entwickleremulsion

| Inhaltsstoff | Menge (Gew.-%) |
|---|---|
| Paraffinum Liquidum (Mineral Oil) | 17 |
| Wasserstoffperoxid | 6,0 |
| Cetearylalkohol | 3,5 |
| PEG-40 Castor Oil | 0,7 |
| Natriumcetearylsulfat | 0,5 |
| Etidronsäure | 0,2 |
| Kaliumhydroxid | 0,1 |
| Dinatriumpyrophosphat | 0,1 |
| Dipicolinsäure (2,6-Dicarboxypyridin) | 0,1 |
| Natriumbenzoat | 0,1 |
| Wasser | ad 100 |

Die jeweilige Blondierpaste und die Entwickleremulsion wurden im Gewichtsverhältnis 1:2 miteinander vermischt.

### 2. Anwendung

100 g der frisch hergestellten Mischung aus der jeweiligen Blondierpaste und die Entwickleremulsion wurden auf trockene Haarsträhnen appliziert (4 g Anwendungsmischung pro Gramm Haar).

Nachdem die Strähnen 45 Minuten lang bei 32 °C blondiert wurden, wurden sie 2 Minuten lang mit Wasser ausgewaschen und mit einem Föhn getrocknet.

Dieser Blondiervorgang wurde einmal wiederholt, so dass die Strähnen insgesamt zweimal nacheinander blondiert wurden.

### 3. Messungen der Haarzugfestigkeit

### Hintergrund

Der Young-Modul ist auch bekannt als Elastizitätsmodul (E-Modul). Er entspricht dem Verhältnis von Spannung ("stress") zu Dehnung ("strain") bei linear elastischem Verhalten (in der Hooke'schen Region).

Das Hooke'sche Elastizitätsgesetz besagt, dass die longitudinale Änderung eines Körpers (Dehnung oder "strain") linear abhängig ist von der Kraft, die die Deformation bewirkt (Spannung oder "stress").
Für feuchtes Haar besteht die lineare Korrelation für eine Dehnung von 0 bis 2%.
Der Young-Modul ist ein Maß für die Stärke einer Faser (je höher der Wert für den Young-Modul, desto stärker ist die Faser).

Die für die Messungen verwendeten Strähnen bestehen aus 40 Fasern (Kerling International (Backnang, Germany), European Natural Hair 7/0; Charge #2014, Mischung 138).

Zunächst wird die mittlere Querschnittfläche jedes einzelnen Haars bestimmt (Universal-Dimensions-Measuring-Device UDM 5000A, (Zimmer GmbH, Darmstadt)), und zwar bei einer Temperatur von 22 °C und einer relative Luftfeuchtigkeit von 50%. Diese Werte werden für die Berechnung der Spannungswerte ("stress values") benötigt.

### 3.2. Bestimmung des Young-Moduls vor der Applikation des Blondiermittels

Alle Haarfasern wurden eine Stunde lang in Wasser eingeweicht. Anschließend wurden sie mit dem Stress-Strain-System MTT 680 mit Kontrolleinheit UV 1000 (Dia-Stron Ltd, UK) mit einer konstanten Geschwindigkeit von 10 mm/min innerhalb der elastischen Phase gedehnt (0-1.5% Verlängerung). Anschließend wurde der E-Modul (Young-Modul) berechnet (Software: UvWin 1.32.1000 (Dia-Stron Ltd, UK).

### 3.3. Bestimmung des Young-Moduls nach der Applikation des Blondiermittels

Nach den vier Blondierbehandlungen wurden die Haarfasern mindestens 48 Stunden lang gelagert.

Die Haarfasern wurden eine Stunde lang in Wasser eingeweicht. Anschließend wurden sie mit dem Stress-Strain-System MTT 680 mit Kontrolleinheit UV 1000 (Dia-Stron Ltd, UK) mit einer konstanten Geschwindigkeit von 10 mm/min innerhalb der elastischen Phase gedehnt (0-1.5% Verlängerung). Anschließend wurde der E-Modul (Young-Modul) berechnet (Software: UvWin 1.32.1000 (Dia-Stron Ltd, UK).

Blondierungsbehandlungen führen zu einem Verlust der Haarfaser an Zugfestigkeit. Mit den erfindungsgemäßen Zusammensetzungen kann dieser Zugfestigkeitsverlust allerdings statistisch signifikant verringert werden.

## Patentansprüche

1. Blondierpaste, enthaltend
a) mindestens ein Oxidationsmittel, ausgewählt aus Natriumpercarbonaten und anorganischen Salzen einer Peroxoschwefelsäure, und sowie Mischungen hiervon,
b) weiterhin eine Dicarbonsäure mit 2 bis 10 Kohlenstoffatomen, ausgewählt aus Bernsteinsäure und/oder mindestens einem Salz der Bernsteinsäure,
c) weiterhin Arginin und Lysin oder mindestens eines der Salze dieser Aminosäuren,
d) mindestens ein Öl in einer Gesamtmenge von 16-60 Gew.-%, bevorzugt 20 - 50 Gew.-%, besonders bevorzugt 25 - 45 Gew.-%, jeweils bezogen auf das Gewicht der Blondierpaste, und
e) 0 bis 4 Gew.-% Wasser, bezogen auf das Gewicht der Blondierpaste.

2. Blondierpaste gemäß Anspruch 1, **dadurch gekennzeichnet, dass** das mindestens eine Oxidationsmittel a) in einer Gesamtmenge von 2,5 bis 65 Gew.-%, bevorzugt 10 bis 60 Gew.-%, weiter bevorzugt 20 bis 55 Gew.-%, besonders bevorzugt 25 bis 50 Gew.-% und insbesondere 30 bis 45 Gew.-%, jeweils bezogen auf das Gewicht der Blondierpaste, enthalten ist.

3. Blondierpaste gemäß Anspruch 1 oder 2, **dadurch gekennzeichnet, dass** Bernsteinsäure und/oder mindestens ein Salz der Bernsteinsäure in einer auf die Masse an freier Dicarbonsäure umgerechneten Gesamtmenge von 0,03 - 7 Gew.-%, bevorzugt 0,1 - 5 Gew.- %, besonders bevorzugt 0,5 - 3 Gew.-%, außerordentlich bevorzugt 0,9 -1,5 Gew.-%, jeweils bezogen auf das Gewicht der Blondierpaste, enthalten ist,
wobei der Gesamtgehalt an Dicarbonsäuren mit 2 bis 10 Kohlenstoffatomen, ausgewählt aus Bernsteinsäure, Äpfelsäure, Oxalsäure, Malonsäure, Adipinsäure, Pimelinsäure, Korksäure, Azelainsäure, Sebacinsäure, Maleinsäure, Fumarsäure, D-Weinsäure, L-Weinsäure, meso-Weinsäure, Traubensäure, alpha-Ketoglutarsäure, beta-Ketoglutarsäure, Oxalessigsäure und den Salzen dieser Säuren, in einer auf die Masse an freier Dicarbonsäure umgerechneten Gesamtmenge 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Blondierpaste, beträgt.

4. Blondierpaste gemäß einem der Ansprüche 1 - 3, **dadurch gekennzeichnet, dass** Arginin und Lysin oder mindestens ein Salz dieser Aminosäuren in einer auf die Masse an freier Aminosäure umgerechneten Gesamtmenge von 0,1 - 7 Gew.-%, bevorzugt 0,2 - 5 Gew.-%, besonders bevorzugt 0,5 - 2,5 Gew.-%, außerordentlich bevorzugt 1 - 2 Gew.-%, jeweils bezogen auf das Gewicht der Blondierpaste, enthalten sind, wobei der Gesamtgehalt an den Aminosäuren Arginin und Lysin, ggf. Histidin, und den Salzen dieser Aminosäuren in einer auf die Masse an freier Aminosäure umgerechneten Gesamtmenge 0,1 - 7 Gew.-%, bevorzugt 0,2 - 5 Gew.-%, besonders bevorzugt 0,5 - 2,5 Gew.-%, außerordentlich bevorzugt 1 - 2 Gew.- %, jeweils bezogen auf das Gewicht der Blondierpaste, beträgt.

5. Blondierpaste gemäß einem der Ansprüche 1 - 4, **dadurch gekennzeichnet, dass** zusätzlich mindestens ein anorganisches, bei 20 °C und 1013 mbar festes Alkalisierungsmittel enthalten ist, das bevorzugt in einer Gesamtmenge von 0,5 - 15 Gew.-%, bevorzugt 1-10 Gew.-%, besonders bevorzugt 2-8 Gew.%, außerordentlich bevorzugt 3-7 Gew.-%, jeweils bezogen auf das Gewicht der Blondierpaste, enthalten ist.

6. Blondierpaste gemäß einem der Ansprüche 1 - 5, **dadurch gekennzeichnet, dass** das mindestens eine Oxidationsmittel a) ausgewählt ist aus Natriumperoxodisulfat, Kaliumperoxodisulfat, Ammoniumperoxodisulfat, Natriumpercarbonat, Natriumperoxomonosulfat, Kaliumperoxomonosulfat, Ammoniumperoxomonosulfat, sowie Mischungen dieser Verbindungen, bevorzugt Mischungen aus Kaliumperoxodisulfat und Ammoniumperoxodisulfat oder Mischungen aus Natriumperoxodisulfat und Ammoniumperoxodisulfat.

7. Blondierpaste gemäß einem der Ansprüche 1-6, **dadurch gekennzeichnet, dass** mindestens eine Substanz, die die Ölphase verdickt, in einer Gesamtmenge von 1 - 15 Gew.-%, bevorzugt 2-10 Gew.-%, besonders bevorzugt 3-8 Gew.-%, besonders bevorzugt 4 - 6,5 Gew.-%, jeweils bezogen auf das Gewicht der Blondierpaste, enthalten ist.

8. Blondierpaste gemäß einem der Ansprüche 1 - 7, **dadurch gekennzeichnet, dass** mindestens eine Substanz, die die Ölphase verdickt, enthalten ist, wobei die mindestens eine Substanz, die die Ölphase verdickt, ausgewählt ist aus Copolymeren von C2-C4-Alken und Styrol, linearen gesättigten 1-Alkanolen mit 12-30 Kohlenstoffatomen, Estern aus gesättigten verzweigten oder unverzweigten Alkancarbonsäuren mit 12 bis 24 C-Atomen und gesättigten verzweigten oder unverzweigten Alkoholen mit 16 bis 50 C-Atomen, wobei die Ester einen Schmelzpunkt im Bereich von 50°C bis 110°C aufweisen, Triglyceriden gesättigter und gegebenenfalls hydroxylierter C₁₂₋₃₀-Fettsäuren, wobei die Triglyceride einen Schmelzpunkt im Bereich von 50°C bis 110°C aufweisen, sowie Mischungen der vorgenannten Substanzen.

9. Verfahren zur Aufhellung von keratinischen Fasern, insbesondere menschlichen Haaren, das **dadurch gekennzeichnet ist, dass** eine Blondierpaste nach einem der Ansprüche 1 - 8 mit einer Oxidationszusammensetzung vermischt wird, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden,
wobei die Blondierpaste (B) und die Oxidationszusammensetzung (Ox) bevorzugt in einem gewichtsbezogenen Verhältnis (B) : (Ox) von 0,2 - 1, besonders bevorzugt 0,3 - 0,8, weiter bevorzugt 0,4 - 0,7, außerordentlich bevorzugt 0,5 - 0,6, miteinander vermischt werden.

10. Mehrkomponentenverpackungseinheit (Kit-of-Parts) zur Aufhellung von keratinischen Fasern, insbesondere menschlichen Haaren, enthaltend mindestens zwei getrennt voneinander verpackte Komponenten, **dadurch gekennzeichnet, dass**
i) die erste Komponente (I) eine Blondierpaste nach einem der Ansprüche 1 - 8 ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei die Komponenten (I) und (II) bevorzugt in einem gewichtsbezogenen Verhältnis (I) : (II) von (0,7 - 1,3) : (2 - 3) : besonders bevorzugt (0,8 -1 ,2) : (2,3 - 2,7), außerordentlich bevorzugt 1:2, zueinander vorliegen.

11. Verfahren zur Farbveränderung von keratinischen Fasern, insbesondere menschlichen Haaren, bei dem eine Blondierpaste nach einem der Ansprüche 1 - 8 mit einer Oxidationszusammensetzung (Ox), die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid und weiterhin mindestens ein pH-Stellmittel in einer solchen Menge enthält, dass die Oxidationszusammensetzung einen pH-Wert im Bereich von 2,5 bis 5,5, gemessen bei 20°C, aufweist,
und zusätzlich mit einer Alkalisierungszusammensetzung (Alk), die Wasser und mindestens ein Alkalisierungsmittel, das ausgewählt ist aus Ammoniak, Alkanolaminen und Mischungen hiervon, enthält und einen pH-Wert im Bereich von 8 - 12, bevorzugt von 9 - 11, besonders bevorzugt von 9,5 - 10,5, aufweist, jeweils gemessen bei 20°C,
vermischt wird,
unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden,
wobei die Blondierpaste (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3 - 2,7) : (2,3 - 2,7), außerordentlich bevorzugt 1:2:2, miteinander vermischt werden.

12. Mehrkomponentenverpackungseinheit (Kit-of-Parts) zur Farbveränderung von keratinischen Fasern, insbesondere menschlichen Haaren, enthaltend mindestens drei getrennt voneinander verpackte Komponenten, **dadurch gekennzeichnet, dass**
i) die erste Komponente (I) eine Blondierpaste nach einem der Ansprüche 1 - 8 ist,
ii) die zweite Komponente (II) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.- %, Wasser und 0,5 - 20 Gew.-% Wasserstoffperoxid enthält und einen pH-Wert im Bereich von 2,5 bis 5,5, gemessen bei 20°C, aufweist,
iii) die dritte Komponente (III) eine Alkalisierungszusammensetzung (Alk) ist, die Wasser und mindestens ein Alkalisierungsmittel, das ausgewählt ist aus Ammoniak, Alkanolaminen und Mischungen hiervon, enthält und einen pH-Wert im Bereich von 8-12, bevorzugt von 9-11, besonders bevorzugt von 9,5 - 10,5, aufweist, jeweils gemessen bei 20°C
wobei die Blondierpaste (B), die Oxidationszusammensetzung (Ox) und die Alkalisierungszusammensetzung (Alk) bevorzugt in einem gewichtsbezogenen Verhältnis (B) : (Ox) : (Alk) von (0,7 - 1,3) : (2 - 3) : (2 - 3), besonders bevorzugt (0,8 - 1,2) : (2,3 - 2,7) : (2,3 - 2,7), außerordentlich bevorzugt 1:2:2, zueinander vorliegen.

13. Verwendung von Bernsteinsäure und/oder mindestens einem Salz der Bernsteinsäure in Kombination mit
Arginin und Lysin oder mindestens einem der Salze dieser Aminosäuren,
in einer Blondierpaste, die mindestens ein Oxidationsmittel, ausgewählt aus Natriumpercarbonaten und anorganischen Salzen einer Peroxoschwefelsäure, und sowie Mischungen hiervon, und weiterhin 0 bis 4 Gew.-% Wasser, bezogen auf das Gewicht der Blondierpaste, enthält, zur Verringerung der Schädigung an keratinischen Fasern, insbesondere menschlichen Haaren, die bewirkt wird durch die Behandlung dieser Fasern mit einer Mischung aus der Blondierpaste und einer Oxidationszusammensetzung, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 2,5 bis 5,5, gemessen bei 20°C, aufweist,
wobei die genannte Blondierpaste bevorzugt eine Blondierpaste nach einem der Ansprüche 2-8 ist.

## Claims

1. A bleaching paste, containing
a) at least one oxidizing agent selected from sodium percarbonates and inorganic salts of a peroxysulfuric acid, and mixtures thereof,
b) also a dicarboxylic acid having 2 to 10 carbon atoms, selected from succinic acid and/or at least one salt of succinic acid,
c) also arginine and lysine or at least one of the salts of these amino acids,
d) at least one oil in a total amount of from 16-60 wt.%, preferably 20-50 wt.%, particularly preferably 25-45 wt.%, in each case based on the weight of the bleaching paste, and
e) 0 to 4 wt.% water, based on the weight of the bleaching paste.

2. The bleaching paste according to claim 1, **characterized in that** the at least one oxidizing agent a) is contained in a total amount of from 2.5 to 65 wt.%, preferably 10 to 60 wt.%, more preferably 20 to 55 wt.%, particularly preferably 25 to 50 wt.%, and in particular 30 to 45 wt.%, in each case based on the weight of the bleaching paste.

3. The bleaching paste according to claim 1 or 2, **characterized in that** succinic acid and/or at least one salt of succinic acid is contained in a total amount, converted to the mass of free dicarboxylic acid, of from 0.03-7 wt.%, preferably 0.1-5 wt.%, particularly preferably 0.5-3 wt.%, extremely preferably 0.9-1.5 wt.%, in each case based on the weight of the bleaching paste, the total content of dicarboxylic acids having 2 to 10 carbon atoms, selected from succinic acid, malic acid, oxalic acid, malonic acid, adipic acid, pimelic acid, suberic acid, azelaic acid, sebacic acid, maleic acid, fumaric acid, D-tartaric acid, L-tartaric acid, meso-tartaric acid, racemic acid, alpha-ketoglutaric acid, beta-ketoglutaric acid, oxaloacetic acid and the salts of these acids, being contained in a total amount, converted to the mass of free dicarboxylic acid, of from 0.03-7 wt.%, preferably 0.1-5 wt.%, particularly preferably 0.5-3 wt.%, and extremely preferably 0.9-1.5 wt.%, in each case based on the weight of the bleaching paste.

4. The bleaching paste according to one of claims 1-3, **characterized in that** arginine and lysine or at least one salt of these amino acids are contained in a total amount, converted to the mass of free amino acid, of from 0.1-7 wt.%, preferably 0.2-5 wt.%, particularly preferably 0.5-2.5 wt.%, extremely preferably 1-2 wt.%, in each case based on the weight of the bleaching paste, the total content of amino acids arginine and lysine, possibly histidine, and the salts of these amino acids being contained in a total amount, converted to the mass of free amino acid, of from 0.1-7 wt.%, preferably 0.2-5 wt.%, particularly preferably 0.5-2.5 wt.%, extremely preferably 1-2 wt.%, in each case based on the weight of the bleaching paste.

5. The bleaching paste according to one of claims 1-4, **characterized in that** at least one inorganic alkalizing agent is additionally contained that is solid at 20 °C and 1013 mbar and is preferably contained in a total amount of from 0.5-15 wt.%, preferably 1-10 wt.%, particularly preferably 2-8 wt.%, extremely preferably 3-7 wt.%, in each case based on the weight of the bleaching paste.

6. The bleaching paste according to one of claims 1-5, **characterized in that** the at least one oxidizing agent a) is selected from sodium peroxydisulfate, potassium peroxydisulfate, ammonium peroxydisulfate, sodium percarbonate, sodium peroxymonosulfate, potassium peroxymonosulfate, ammonium peroxymonosulfate and mixtures of these compounds, preferably mixtures of potassium peroxydisulfate and ammonium peroxydisulfate or mixtures of sodium peroxydisulfate and ammonium peroxydisulfate.

7. The bleaching paste according to one of claims 1-6, **characterized in that** at least one substance which thickens the oil phase is contained in a total amount of from 1-15 wt.%, preferably 2-10 wt.%, particularly preferably 3-8 wt.%, particularly preferably 4-6.5 wt.%, in each case based on the weight of the bleaching paste.

8. The bleaching paste according to one of claims 1-7, **characterized in that** at least one substance which thickens the oil phase is contained, the at least one substance which thickens the oil phase being selected from copolymers of C2-C4 alkene and styrene, linear saturated 1-alkanols having 12-30 carbon atoms, esters of saturated branched or unbranched alkane carboxylic acids having 12 to 24 C atoms, and saturated branched or unbranched alcohols having 16 to 50 C atoms, the esters having a melting point in the range of from 50 °C to 110 °C, triglycerides of saturated and optionally hydroxylated C₁₂₋₃₀ fatty acids, the triglycerides having a melting point in the range of from 50 °C to 110 °C, and mixtures of the aforementioned substances.

9. A method for lightening keratin fibers, in particular human hair, which method is **characterized in that** a bleaching paste according to one of claims 1-8 is mixed with an oxidizing composition, which contains, in each case based on its weight, 50-96 wt.%, preferably 70-93 wt.%, particularly preferably 80-90 wt.%, water and 0.5-20 wt.% hydrogen peroxide and also contains at least one pH adjuster in such an amount that the oxidizing composition has a pH in the range of from 2.5 to 5.5, measured at 20 °C, and immediately thereafter is applied to the keratin fibers and left on the fibers for 5 to 60 minutes and subsequently the fibers are rinsed with water and optionally washed with a surfactant-containing cleaning agent, the bleaching paste (B) and the oxidizing composition (Ox) preferably being mixed with one another in a weight-based ratio (B):(Ox) of 0.2-1, particularly preferably 0.3-0.8, more preferably 0.4-0.7, extremely preferably 0.5-0.6.

10. A multi-component packaging unit (kit-of-parts) for lightening keratin fibers, in particular human hair, containing at least two components packaged separately from one another, **characterized in that**
i) the first component (I) is a bleaching paste according to one of claims 1-8,
ii) the second component (II) is an oxidizing composition which contains, in each case based on its weight, 50-96 wt.% preferably 70-93 wt.%, particularly preferably 80-90 wt.%, water and 0.5-20 wt.% hydrogen peroxide and has a pH in the range of from 2.5 to 5.5, measured at 20 °C,
wherein components (I) and (II) are preferably in a weight-based ratio (I):(II) to one another of (0.7-1.3):(2-3), particularly preferably (0.8-1.2):(2.3-2.7), extremely preferably 1:2.

11. A method for changing the color of keratin fibers, in particular human hair, in which a bleaching paste according to one of claims 1-8 is mixed with an oxidizing composition (Ox) which contains, in each case based on its weight, 50-96 wt.%, preferably 70-93 wt.%, particularly preferably 80-90 wt.%, water and 0.5-20 wt.% hydrogen peroxide and also at least one pH adjuster in such an amount that the oxidizing composition has a pH in the range of from 2.5 to 5.5, measured at 20 °C, and additionally with an alkalizing composition (Alk) which contains water and at least one alkalizing agent, selected from ammonia, alkanolamines and mixtures thereof, and has a pH in the range of from 8-12, preferably 9-11, particularly preferably 9.5-10.5, in each case measured at 20 °C, and immediately thereafter is applied to the keratin fibers and left on the fibers for 5 to 60 minutes and subsequently the fibers are rinsed with water and optionally washed with a surfactant-containing cleaning agent, wherein the bleaching paste (B), the oxidizing composition (Ox) and the alkalizing composition (Alk) are preferably mixed together in a weight-based ratio (B):(Ox):(Alk) of (0.7-1.3):(2-3):(2-3), particularly preferably (0.8-1.2):(2.3-2.7):(2.3-2.7), extremely preferably 1:2:2.

12. A multi-component packaging unit (kit-of-parts) for changing the color of keratin fibers, in particular human hair, containing at least three components packaged separately from one another, **characterized in that**
i) the first component (I) is a bleaching paste according to one of claims 1-8,
ii) the second component (II) is an oxidizing composition which contains, in each case based on its weight, 50-96 wt.% preferably 70-93 wt.%, particularly preferably 80-90 wt.%, water and 0.5-20 wt.% hydrogen peroxide and has a pH in the range of from 2.5 to 5.5, measured at 20 °C,
iii) the third component (III) is an alkalizing composition (Alk) which contains water and at least one alkalizing agent, which is selected from ammonia, alkanolamines and mixtures thereof, and has a pH in the range of from 8-12, preferably from 9-11, particularly preferably from 9.5-10.5, in each case measured at 20 °C,
wherein the bleaching paste (B), the oxidizing composition (Ox) and the alkalizing composition (Alk) are preferably in a weight-based ratio (B):(Ox):(Alk) to one another of (0.7-1.3):(2-3):(2-3), particularly preferably (0.8-1.2):(2.3-2.7):(2.3-2.7), extremely preferably 1:2:2.

13. The use of succinic acid and/or at least one salt of succinic acid in combination with arginine and lysine or at least one of the salts of these amino acids in a bleaching paste which contains at least one oxidizing agent, selected from sodium percarbonates and inorganic salts of a peroxysulfuric acid and mixtures thereof, and also 0 to 4 wt.% water, based on the weight of the bleaching paste, for reducing damage to keratin fibers, in particular human hair, caused by the treatment of these fibers with a mixture of the bleaching paste and an oxidizing composition which, in each case based on its weight, contains 50-96 wt.%, preferably 70-93 wt.%, particularly preferably 80-90 wt.%, water and 0.5-20 wt.% hydrogen peroxide and has a pH in the range of from 2.5 to 5.5, measured at 20 °C, the above-mentioned bleaching paste preferably being a bleaching paste according to one of claims 2-8.

## Revendications

1. Pâte décolorante, contenant
a) au moins un agent oxydant choisi parmi les percarbonates de sodium et les sels inorganiques d'un acide peroxosulfurique, et leurs mélanges,
b) également un acide dicarboxylique comportant 2 à 10 atomes de carbone, choisi parmi l'acide succinique et/ou au moins un sel de l'acide succinique,
c) également de l'arginine et de la lysine ou au moins l'un des sels de ces acides aminés,
d) au moins une huile en une quantité totale de 16 à 60% en poids, de préférence de 20 à 50% en poids, de manière particulièrement préférée de 25 à 45% en poids, respectivement par rapport au poids de la pâte décolorante, et
e) 0 à 4 % en poids d'eau, par rapport au poids de la pâte décolorante.

2. Pâte décolorante selon la revendication 1, **caractérisée en ce qu'**elle contient l'au moins un agent oxydant a) en une quantité totale de 2,5 à 65 % en poids, de préférence de 10 à 60 % en poids, plus préférablement de 20 à 55 % en poids, de manière particulièrement préférée de 25 à 50 % en poids et en particulier de 30 à 45 % en poids, respectivement par rapport au poids de la pâte décolorante.

3. Pâte décolorante selon la revendication 1 ou 2, **caractérisée en ce qu'**elle contient de l'acide succinique et/ou au moins un sel de l'acide succinique en une quantité totale, convertie en la masse d'acide dicarboxylique libre, de 0,03 à 7 % en poids, de préférence de 0,1 à 5 % en poids, de manière particulièrement préférée de 0,5 à 3 % en poids, encore plus préférablement de 0,9 à 1,5 % en poids, respectivement par rapport au poids de la pâte décolorante,
la teneur totale en acides dicarboxyliques comportant 2 à 10 atomes de carbone, choisis parmi l'acide succinique, l'acide malique, l'acide oxalique, l'acide malonique, l'acide adipique, l'acide pimélique, l'acide subérique, l'acide azélaïque, l'acide sébacique, l'acide maléique, l'acide fumarique, l'acide D-tartrique, l'acide L-tartrique, l'acide méso-tartrique, l'acide racémique, l'acide alpha-cétoglutarique, l'acide bêta-cétoglutarique, l'acide oxaloacétique et les sels de ces acides, en une quantité totale convertie en la masse d'acide dicarboxylique libre, étant de 0,03 à 7 % en poids, de préférence de 0,1 à 5 % en poids, de manière particulièrement préférée de 0,5 à 3 % en poids, encore plus préférablement de 0,9 à 1,5 % en poids, respectivement par rapport au poids de la pâte décolorante.

4. Pâte décolorante selon l'une des revendications 1 à 3, **caractérisée en ce qu'**elle contient de l'arginine et de la lysine ou au moins un sel de ces acides aminés en une quantité totale, convertie en la masse d'acide aminé libre, de 0,1 à 7 % en poids, de préférence de 0,2 à 5 % en poids, de manière particulièrement préférée de 0,5 à 2,5 % en poids, encore plus préférablement de 1 à 2 % en poids, respectivement par rapport au poids de la pâte décolorante, la teneur totale des acides aminés arginine et lysine, éventuellement histidine, ou des sels de ces acides aminés, en une quantité totale convertie en la masse d'acide aminé libre, étant de 0,1 à 7 % en poids, de préférence de 0,2 à 5 % en poids, de manière particulièrement préférée de 0,5 à 2,5 % en poids, encore plus préférablement de 1 à 2 % en poids, respectivement par rapport au poids de la pâte décolorante.

5. Pâte décolorante selon l'une des revendications 1 à 4, **caractérisée en ce qu'**elle contient en outre au moins un agent alcalinisant inorganique solide à 20 °C et à 1013 mbar, lequel est contenu de préférence en une quantité totale de 0,5 à 15 % en poids, de préférence de 1 à 10 % en poids, de manière particulièrement préférée de 2 à 8 % en poids, encore plus préférablement de 3 à 7 % en poids, respectivement par rapport au poids de la pâte décolorante.

6. Pâte décolorante selon l'une des revendications 1 à 5, **caractérisée en ce que** l'au moins un agent oxydant a) est choisi parmi le peroxodisulfate de sodium, le peroxodisulfate de potassium, le peroxodisulfate d'ammonium, le percarbonate de sodium, le peroxomonosulfate de sodium, le peroxomonosulfate de potassium, le peroxomonosulfate d'ammonium, et des mélanges de ces composés, de préférence des mélanges de peroxodisulfate de potassium et de peroxodisulfate d'ammonium ou des mélanges de peroxodisulfate de sodium et de peroxodisulfate d'ammonium.

7. Pâte décolorante selon l'une des revendications 1 à 6, **caractérisée en ce qu'**elle contient au moins une substance, laquelle épaissit la phase huileuse, en une quantité totale de 1 à 15 % en poids, de préférence de 2 à 10 % en poids, de manière particulièrement préférée de 3 à 8 % en poids, de manière plus préférée de 4 à 6,5 % en poids, respectivement par rapport au poids de la pâte décolorante.

8. Pâte décolorante selon l'une des revendications 1 à 7, **caractérisée en ce qu'**elle contient au moins une substance, laquelle épaissit la phase huileuse, l'au moins une substance qui épaissit la phase huileuse étant choisie parmi les copolymères d'alcène en C2-C4 et de styrène, les 1-alcanols linéaires saturés comportant 12 à 30 atomes de carbone, les esters d'acides alcanecarboxyliques saturés ramifiés ou non ramifiés comportant 12 à 24 atomes de carbone et les alcools saturés ramifiés ou non ramifiés comportant 16 à 50 atomes de carbone, les esters présentant un point de fusion compris entre 50 °C et 110 °C, les triglycérides d'acides gras saturés et éventuellement hydroxylés en C₁₂₋₃₀, les triglycérides présentant un point de fusion compris entre 50 °C et 110 °C, et des mélanges des substances précitées.

9. Procédé d'éclaircissement de fibres kératiniques, en particulier de cheveux humains, **caractérisé en ce qu'**une pâte décolorante selon l'une des revendications 1 à 8 est mélangée à une composition oxydante qui, respectivement par rapport à son poids, contient 50 à 96 % en poids, de préférence 70 à 93 % en poids, de manière particulièrement préférée 80 à 90 % en poids d'eau et 0,5 à 20 % en poids de peroxyde d'hydrogène et également au moins un agent d'ajustement du pH en une quantité telle que la composition oxydante présente un pH compris entre 2,5 et 5,5, mesuré à 20 °C, qu'elle est appliquée immédiatement après sur les fibres contenant de la kératine, y est laissée pendant 5 à 60 minutes, puis que les fibres sont rincées à l'eau et éventuellement lavées avec un agent nettoyant contenant un tensioactif,
la pâte décolorante (B) et la composition oxydante (Ox) étant mélangées l'une à l'autre de préférence dans un rapport pondéral (B) : (Ox) de 0,2 à 1, de manière particulièrement préférée de 0,3 à 0,8, de manière davantage préférée de 0,4 à 0,7, encore plus préférablement de 0,5 à 0,6.

10. Unité de conditionnement multi-constituant (kit de pièces) permettant l'éclaircissement de fibres kératiniques, en particulier de cheveux humains, contenant au moins deux constituants conditionnés séparément les uns des autres, **caractérisée en ce que**
i) le premier constituant (I) est une pâte décolorante selon l'une des revendications 1 à 8,
ii) le deuxième constituant (II) est une composition oxydante qui présente, respectivement par rapport à son poids, 50 à 96 % en poids, de préférence 70 à 93 % en poids, de manière particulièrement préférée 80 à 90 % en poids d'eau et 0,5 à 20 % en poids de peroxyde d'hydrogène, et un pH compris entre 2,5 et 5,5, mesuré à 20 °C,
les constituants (I) et (II) étant présents l'un par rapport à l'autre de préférence dans un rapport pondéral (I) : (II) de (0,7 à 1,3) : (2 à 3), de manière particulièrement préférée de (0,8 à 1,2) : (2,3 à 2,7), de préférence 1:2.

11. Procédé permettant la coloration de fibres kératiniques, en particulier de cheveux humains, selon lequel une pâte décolorante selon l'une des revendications 1 à 8 est mélangée à une composition oxydante (Ox) qui, respectivement par rapport à son poids, contient 50 à 96 % en poids, de préférence 70 à 93 % en poids, de manière particulièrement préférée 80 à 90 % en poids d'eau et 0,5 à 20 % en poids de peroxyde d'hydrogène et également au moins un agent d'ajustement du pH en une quantité telle que la composition oxydante présente un pH compris entre 2,5 et 5,5, mesuré à 20 °C,
et est mélangée en outre à une composition alcalinisante (Alk), qui contient de l'eau et au moins un agent alcalinisant choisi parmi l'ammoniac, les alcanolamines et leurs mélanges, et qui présente un pH compris entre 8 et 12, de préférence entre 9 et 11, de manière particulièrement préférée entre 9,5 et 10,5, mesuré respectivement à 20 °C,
est appliquée immédiatement après sur les fibres kératiniques, y est laissée pendant 5 à 60 minutes, puis les fibres sont rincées à l'eau et éventuellement lavées avec un agent nettoyant contenant un tensioactif,
la pâte décolorante (B), la composition oxydante (Ox) et la composition alcalinisante (Alk) étant mélangées les unes aux autres de préférence dans un rapport pondéral (B) : (Ox) : (Alk) de (0,7 à 1,3) : (2 à 3) : (2 à 3), de manière particulièrement préférée de (0,8 à 1,2) : (2,3 à 2,7) : (2,3 à 2,7), encore plus préférablement 1:2:2.

12. Unité de conditionnement multi-constituant (kit de pièces) permettant la coloration de fibres kératiniques, en particulier de cheveux humains, contenant au moins trois constituants conditionnés séparément les uns des autres, **caractérisée en ce que**
i) le premier constituant (I) est une pâte décolorante selon l'une des revendications 1 à 8,
ii) le deuxième constituant (II) est une composition oxydante qui présente, respectivement par rapport à son poids, 50 à 96 % en poids, de préférence 70 à 93 % en poids, de manière particulièrement préférée 80 à 90 % en poids d'eau et 0,5 à 20 % en poids de peroxyde d'hydrogène, et un pH compris entre 2,5 et 5,5, mesuré à 20 °C,
iii) le troisième constituant (III) est une composition alcalinisante (Alk) contenant de l'eau et au moins un agent alcalinisant choisi parmi l'ammoniac, les alcanolamines et leurs mélanges, et présentant un pH compris entre 8 et 12, de préférence entre 9 et 11, de manière particulièrement préférée entre 9,5 et 10,5, mesuré respectivement à 20 °C,
la pâte décolorante (B), la composition oxydante (Ox) et la composition alcalinisante (Alk) étant présentes les unes par rapport aux autres de préférence dans un rapport pondéral (B) : (Ox) : (Alk) de (0,7 à 1,3) : (2 à 3) : (2 à 3), de manière particulièrement préférée de (0,8 à 1,2) : (2,3 à 2,7) : (2,3 à 2,7), encore plus préférablement de 1:2:2.

13. Utilisation d'acide succinique et/ou d'au moins un sel de l'acide succinique en association avec
de l'arginine et de la lysine ou au moins de l'un des sels de ces acides aminés,
dans une pâte décolorante qui contient au moins un agent oxydant choisi parmi les percarbonates de sodium et les sels inorganiques d'un acide peroxosulfurique, ainsi que leurs mélanges, et qui contient en outre 0 à 4 % en poids d'eau, par rapport au poids de la pâte décolorante, pour réduire les dommages causés aux fibres kératiniques, en particulier aux cheveux humains, par le traitement de ces fibres avec un mélange de la pâte décolorante et d'une composition oxydante, qui, respectivement par rapport à son poids, contient de 50 à 96 % en poids, de préférence de 70 à 93 % en poids, de manière particulièrement préférée de 80 à 90 % en poids d'eau et de 0,5 à 20 % en poids de peroxyde d'hydrogène, et qui présente un pH compris entre 2,5 et 5,5, mesuré à 20 °C,
ladite pâte décolorante étant de préférence une pâte décolorante selon l'une des revendications 2 à 8.
